(19) Europäisches Patentamt
European Patent Office
Office européen des brevets

(11) Veröffentlichungsnummer: **0 071 571**
**B1**

(12) # EUROPÄISCHE PATENTSCHRIFT

(45) Veröffentlichungstag der Patentschrift: **04.09.85**

(51) Int. Cl.⁴: **G 03 C 1/68,** G 03 F 7/10

(21) Anmeldenummer: **82810311.9**

(22) Anmeldetag: **19.07.82**

(54) **Photopolymerisationsverfahren.**

(30) Priorität: **23.07.81 GB 8122803**

(43) Veröffentlichungstag der Anmeldung:
**09.02.83 Patentblatt 83/6**

(45) Bekanntmachung des Hinweises auf die Patenterteilung:
**04.09.85 Patentblatt 85/36**

(84) Benannte Vertragsstaaten:
**BE CH DE FR GB IT LI NL SE**

(56) Entgegenhaltungen:
**DE - A - 2 445 395**
**US - A - 3 926 642**

**RESEARCH DISCLOSURE, Oktober 1972, Seiten 26,27, Nr. 10234, Industrial Opportunities Ltd., Havant, Hampshire, G.B., "A polymeric photorecording medium"**
**RESEARCH DISCLOSURE, Dezember 1980, Seiten 527-528, Nr. 20012, Industrial Opportunities Ltd., Havant, Hampshire, G.B., "Electron beam resists"**

(73) Patentinhaber: **CIBA-GEIGY AG, Postfach, CH-4002 Basel (CH)**

(72) Erfinder: **Paul, John Gordon, Dr., 13 Alma Terrace, Falkirk Stirlingshire (GB)**
Erfinder: **Stark, Bernard Peter, Dr., 41 High Street, Great Shelford Cambridge CB2 5EH (GB)**
Erfinder: **Losert, Ewald, Habsburgerstrasse 30, CH-4310 Rheinfelden (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Vorliegende Erfindung betrifft ein Photopolymerisations- und Photovernetzungsverfahren sowie insbesondere ein solches Verfahren zur Erzeugung von Abbildungen.

Üblicherweise erfolgt die Erzeugung einer Abbildung durch Photopolymerisation, indem man einen Träger mit einer Lösung einer photopolymerisierbaren Substanz in einem flüchtigen organischen Lösungsmittel beschichtet, das Lösungsmittel abdampft oder verdampfen lässt, so dass ein Film der photopolymerisierbaren Substanz verbleibt, den Film mit aktinischer Strahlung wie durch eine Abbildung hindurch bestrahlt, wodurch die von der Strahlung getroffenen Stellen des Films photopolymerisiert (und weniger löslich) werden, während die von der Strahlung abgeschirmten Stellen weitgehend unverändert bleiben, und dann die unbestrahlten, unphotopolymerisierten Stellen des Films mittels eines geeigneten Lösungsmittels, welches die bestrahlten, photopolymerisierten Stellen nicht auflöst, weglöst. Diese letzte Stufe ist üblicherweise als «Entwicklung» bekannt.

Es wäre wünschenswert, ein Verfahren zur Verfügung zu haben, bei dem eine Schicht einer photopolymerisierbaren Substanz auf einen Träger aufgebracht und diese Schicht ohne Anwendung organischer Lösungsmittel in einen weitgehend festen, klebfreien, bestrahlungsfertigen Zustand überführt würde. In dieser Stufe würde man nicht nur die Anwendung von Lösungsmitteln, die zu Toxizitäts- und Brennbarkeitsproblemen führen könnten und auch Rückgewinnungskosten verursachen, vermeiden, sondern auch die Herstellung beschichteter, bestrahlungsfertiger Träger auf kontinuierliche Weise würde erleichtert werden.

Es wurde nun gefunden, dass man diese Aufgabe dadurch lösen kann, dass man gewisse Substanzen, die im selben Molekül zwei Arten von Gruppen enthalten, über welche Photopolymerisation mit beträchtlich voneinander verschiedenen Geschwindigkeiten eintreten kann, verwendet. Die Gruppen werden so ausgewählt, dass Photopolymerisation einer Schicht einer flüssigen Zusammensetzung zu einer festen, im wesentlichen klebfreien Schicht, die jedoch in bestimmten Lösungsmitteln noch löslich ist, rasch eintritt. Anschliessend setzt man Stellen der Schicht noch einer wesentlich grösseren Menge aktinischer Strahlung aus, wobei Photovernetzung über den anderen Typus von Gruppe in den bereits photopolymerisierten Molekülen der Schicht eintritt und die der Photovernetzung unterworfenen Stellen der Schicht viel beständiger Auflösung in den Lösungsmitteln werden.

In der bekanntgemachten brasilianischen Patentanmeldung Nr. 8 008 428 ist eine Methode zur Erzeugung von Reliefabbildungen aus einem Film eines flüssigen photopolymerisierbaren Materials beschrieben, welche darin besteht, dass man den Film aktinischer Strahlung belichtet, so dass er sich durch chemische Härtung verfestigt, den verfestigten Film dann erneut mit weiterer aktinischer Strahlung in Form eines Musters belichtet, so dass Teile des Films chemisch differenziert werden, und danach die nicht der bemusterten Belichtung mit aktinischer Strahlung unterworfenen Stellen des Films selektiv durch Waschen mit einem Lösungsmittel entfernt. Die einzigen dabei vorgeschlagenen, photopolymerisierbaren Materialien bestehen aus einem Gemisch eines Polyens und eines Polythiols.

Unveröffentlichte Versuche der Anmelderin haben gezeigt, dass zahlreiche möglicherweise nützliche Verbindungen, die zwei Typen normalerweise photopolymerisationsfähiger Einheiten enthalten, bei einem solchen Verfahren keine befriedigenden Ergebnisse liefern, da die Photopolymerisation in der ersten Stufe erheblich verzögert wird, anscheinend als Folge der Gegenwart eines anderen Typs photopolymerisierbarer Einheit im Molekül, trotz des Einbaus verschiedener Photoinitiatoren und Photosensibilisatoren. Weitere unveröffentlichte Versuche der Anmelderin mit Gemischen aus zwei Verbindungen, wovon eine eine normalerweise photopolymerisationsfähige Einheit und die andere eine normalerweise bei Strahlungsbelichtung dimerisationsfähige Einheit enthalten, lieferten unbefriedigende Ergebnisse, offensichtlich wegen Hemmung der Photovernetzungsreaktion.

Es wurde nun gefunden, dass man das gewünschte Verfahren realisieren kann, wenn man eine Verbindung einsetzt, die im selben Molekül sowohl eine oder mehrere Acryloyl- oder Methacryloylgruppen als auch eine oder mehrere Bicyclo[2.2.1]hept-2-en-strukturen enthält.

Ein Gegenstand dieser Erfindung ist dementsprechend ein Verfahren zur Erzeugung einer Abbildung, wobei man (1) eine auf einem Träger aufgebrachte Schicht aus einer flüssigen Zusammensetzung so mit aktinischer Strahlung belichtet, dass sich die Schicht durch Photopolymerisation verfestigt und im wesentlichen klebfrei wird, jedoch weitgehend photovernetzbar bleibt, und anschliessend (2) die so verfestigte Schicht durch eine bildtragende Vorlage mit weitgehend undurchsichtigen und weitgehend durchsichtigen Stellen hindurch mit einer wesentlich grösseren Menge aktinischer Strahlung belichtet, so dass die weiterbelichtete(n) Stelle(n) der photopolymerisierten Schicht erneut Photovernetzung eingehen und (3) die Abbildung durch Auflösung der nicht weitgehend photovernetzten Stellen der Schicht in einem Lösungsmittel entwickelt, welches dadurch gekennzeichnet ist, dass man eine flüssige Zusammensetzung verwendet, die eine Verbindung (A) mit sowohl mindestens einer Gruppe der Formel I

$$\begin{array}{l} CH_2=CCO- \\ \quad\;\; | \\ \quad\;\; R \end{array} \qquad I$$

als auch mindestens einer Gruppe der Formel II

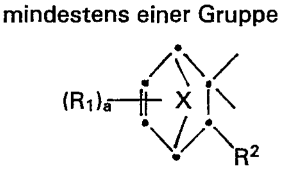

II

im selben Molekül enthält, wobei R ein Wasserstoffatom oder eine Methylgruppe bedeutet, a null oder einer ganze Zahl von 1 bis 4 ist, $R^1$ eine Methyl-

gruppe darstellt oder, falls a 1 ist, auch für eine Allylgruppe stehen kann, $R^2$ ein Wasserstoffatom, eine Carboxylgruppe, eine gegebenenfalls in der Kette durch ein Äthersauerstoffatom unterbrochene Carbonyloxyalkylgruppe, eine über ein Sauerstoffatom an eine Gruppe der Formel I gebundene Carbonyloxyalkylengruppe, wobei die Alkyl- bzw. Alkylengruppe jeweils bis zu 8 Kohlenstoffatome aufweist, oder zusammen mit einer der angegebenen freien Valenzen

-OC
eine cyclische Imidgruppe der Konstitution >N-
-OC

darstellt und X entweder eine gegebenenfalls durch eine oder zwei der oben erwähnten Methylgruppen $R^1$ oder durch die Allylgruppe $R^1$ substituierte Methylenbrücke oder ein Sauerstoffatom bedeutet, wobei die Photopolymerisation von (A) in Verfahrensschritt (1) über die Gruppe bzw. Gruppen der Formel I und die anschliessende Photovernetzung in Verfahrensschritt (2) über die Gruppe bzw. Gruppen der Formel II erfolgt.

In dem Ausdruck «durch eine bildtragende Vorlage mit weitgehend undurchsichtigen und weitgehend durchsichtigen Stellen hindurch belichtet» ist auch Belichtung mit einem sich gemäss einem rechnergesteuerten, vorbestimmten Muster bewegenden Laserstrahl zur Erzeugung einer Abbildung einbegriffen.

Der Einfachheit halber werden mindestens eine Gruppe der Formel II enthaltende Verbindungen hierin als Bicyclo[2.2.1]hept-2-ene bezeichnet, d.h. die 7-Oxaanaloge sind in dem Begriff miteingeschlossen, falls der Zusammenhang dies nicht ausschliesst.

Es ist bekannt, dass Norbornen (d.h. Bicyclo-[2.2.1]hept-2-en) bei Belichtung mit aktinischer Strahlung dimerisiert [Photochemistry (Photochemie), von D.R. Arnold u.a., Verlag Academic Press, 1974, auf Seite 170]. Es ist ebenfalls weithin bekannt, dass Acrylat- und Methacrylatester bei solcher Bestrahlung Photopolymerisation eingehen. Es wird jedoch angenommen, dass die Erzeugung einer Abbildung durch ein zweistufiges Photopolymerisations- und Vernetzungsverfahren mittels einer im selben Molekül mindestens eine Gruppe der Formel I und mindestens eine Gruppe der Formel II aufweisenden Verbindung neu ist.

Üblicherweise wird die im erfindungsgemässen Verfahren eingesetzte Verbindung (A) pro Durchschnittsmolekül bis vier Gruppen der Formel I und vorzugsweise bis vier Gruppen der Formel II enthalten. Im allgemeinen besitzt sie ein Molekulargewicht von höchstens 10 000 und vorzugsweise höchstens 2000. Bevorzugt sind die Gruppe(n) der Formel I sowie vorzugsweise auch die Gruppe(n) der Formel II jeweils direkt an ein Kohlenstoff-, Sauerstoff- oder Stickstoffatom bzw. -atome gebunden.

Zur Verwendung als (A) geeignete Verbindungen mit mindestens einer Gruppe der Formel II, worin $R^2$ für ein Wasserstoffatom steht, lassen sich dadurch erhalten, dass man gleichzeitig oder in beliebiger Reihenfolge eine Verbindung mit zwei oder mehr direkt an ein bzw. mehrere Sauerstoff-, Stickstoff- oder Schwefelatom(e) gebundenen Glycidylgruppen mit Acryl- oder Methacrylsäure und mit einer Bicyclo-[2.2.1]hept-2-en-6-carbonsäure der Formel

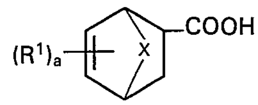 III

worin $R^1$, X und a die oben angegebenen Bedeutungen haben, unter Öffnung der Epoxidringe in den Glycidylgruppen umsetzt.

Somit kann man als Verbindung (A) Substanzen der Formel

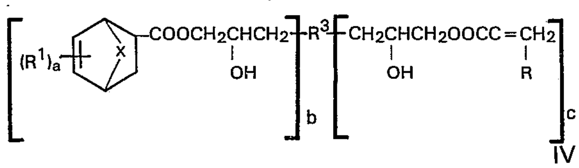
IV

verwenden, worin b und c unabhängig voneinander je eine ganze Zahl von mindestens 1 und vorzugsweise höchstens 4 sind, R, $R^1$, X und a die oben angegebenen Bedeutungen haben und $R^3$ den Rest einer Verbindung mit mindestens (b+c) direkt an ein bzw. mehrere Sauerstoff-, Stickstoff- oder Schwefelatom(e) gebundenen Glycidylgruppen nach Wegnahme von (b+c) solchen Glycidylgruppen darstellt.

Es versteht sich, dass wegen der Konkurrenz für die Epoxidgruppen das Produkt ebenfalls aus der Verbindung mit zwei oder mehr Glycidylgruppen und nur Acrylsäure oder Methacrylsäure bzw. aus der Verbindung mit zwei oder mehr Glycidylgruppen und nur der Bicyclo[2.2.1]hept-2-en-6-carbonsäure gebildete Addukte enthalten wird, d.h. Verbindungen der Formel

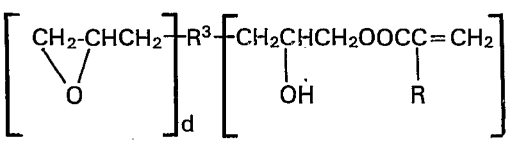 V

sowie Verbindungen der Formel

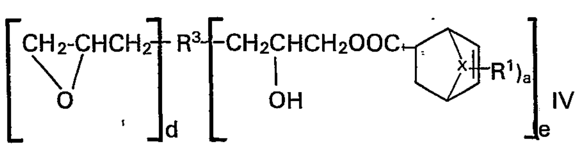 IV

worin d null oder eine positive ganze Zahl und e eine ganze Zahl von mindestens eins sind, wobei die Summe (d+e) der Summe (b+c) gleich ist, und R, $R^1$, $R^3$, X und a die oben angegebenen Bedeutungen haben.

Das Ausmass der Bildung der Nebenprodukte der Formel V oder VI wird natürlich von den Anteilen der drei Arten eingesetzter Reaktionspartner abhängen. Im allgemeinen wird die Durchführung des erfindungsgemässen Verfahrens durch die Gegenwart solcher Nebenprodukte nicht gestört.

Als Beispiele für Glycidylgruppen enthaltende Verbindungen, die mit Acrylsäure oder Methacrylsäure und mit einer Bicyclo[2.2.1]hept-2-en-6-carbonsäure der Formel III behandelt werden können, seien Polyglycidylester genannt, die man durch Umsetzung einer Verbindung mit zwei oder mehr Carbonsäuregruppen pro Molekül mit Epichlorhydrin oder Glycerindichlorhydrin in Gegenwart von Alkali erhalten kann. Solche Polyglycidylester können sich von aliphatischen Polycarbonsäure, z.B. Bernsteinsäure, Glutarsäure, Adipinsäure, Pimelinsäure, Korksäure,

Azelainsäure, Sebacinsäure oder dimerisierter oder trimerisierter Linolsäure, von cycloaliphatischen Polycarbonsäuren wie Tetrahydrophthalsäure, 4-Methyltetrahydrophthalsäure, Hexahydrophthalsäure und 4-Methylhexahydrophthalsäure sowie von aromatischen Polycarbonsäuren wie Phthalsäure, Isophthalsäure und Terephthalsäure ableiten. Weitere geeignete Polyglycidylester sind durch Polymerisation von Glycidylestern von Vinylsäuren, insbesondere Glycidylacrylat und Glycidylmethacrylat, erhältlich.

Weitere Beispiele sind Polyglycidyläther, die durch Umsetzung einer mindestens zwei freie alkoholische und/oder phenolische Hydroxylgruppen pro Molekül enthaltenden Verbindung mit Epichlorhydrin unter alkalischen Bedingungen, oder auch in Gegenwart eines sauren Katalysators mit nachfolgender Alkalibehandlung, erhältlich sind. Diese Äther lassen sich mit Polyepichlorhydrinen aus acyclischen Alkoholen wie Äthylenglykol, Diäthylenglykol und höheren Poly-(oxyäthylen)-glykolen, Propan-1,2-diol und Poly-(oxypropylen)-glykolen, Propan-1,3-diol, Poly-(oxytetramethylen)-glykolen, Pentan-1,5-diol, Hexan-2,4,6-triol, Glycerin, 1,1,1-Trimethylolpropan, Pentaerithrit und Sorbit, aus cycloaliphatischen Alkoholen wie Resorcit, Chinit, Bis-(4-hydroxycyclohexyl)-methan, 2,2-Bis-(4-hydroxycyclohexyl)-propan und 1,1-Bis-(hydroxymethyl)-cyclohexen-3 und aus Alkoholen mit aromatischen Kernen, wie N,N-Bis-(2-hydroxyäthyl)-anilin und p,p'-Bis-(2-hydroxyäthylamino)-diphenylmethan herstellen. Man kann sie ferner aus einkernigen Phenolen, wie Resorcin und Hydrochinon, und mehrkernigen Phenolen wie Bis-(4-hydroxyphenyl)-methan (sonst als Bisphenol F bekannt), 4,4'-Dihydroxydiphenyl, Bis-(4-hydroxyphenyl)-sulfon, 1,1,2,2-Tetrakis-(4-hydroxyphenyl)-äthan, 2,2-Bis-(4-hydroxyphenyl)-propan (sonst als Bisphenol A bekannt) und 2,2-Bis-(3,5-dibrom-4-hydroxyphenyl)-propan sowie aus Aldehyden wie Formaldehyd, Acetaldehyd, Chloral und Furfurol, mit Phenolen wie Phenol selbst und durch Chloratome oder Alkylgruppen mit jeweils bis zu neuen Kohlenstoffatomen ringsubstituiertem Phenol wie 4-Chlorphenol, 2-Methylphenol und 4-tert.-Butylphenol gebildeten Novolaken erhalten.

Poly-(N-glycidyl)-verbindungen sind ebenfalls verwendbar, z.B. N-Glycidylderivate von Aminen wie Anilin, n-Butylamin, Bis-(4-aminophenyl)-methan und Bis-(4-methylaminophenyl)-methan, Triglycidylisocyanurat sowie N,N'-Diglycidylderivate von cyclischen Alkylenharnstoffen, wie Äthylenharnstoff und 1,3-Propylenharnstoff, und Hydantoinen wie 5,5-Dimethylhydantoin.

Auch kann man Poly-(S-glycidyl)-verbindungen einsetzen, z.B. Di-(S-glycidyl)-derivate von Dithiolen wie Äthan-1,2-dithiol und Bis(4-mercaptomethylphenyl)-äther, doch sind diese nicht bevorzugt.

In Betracht kommen auch Polyepoxide, in welchen die 1,2-Epoxidgruppen an Heteroatome verschiedener Art gebunden sind, beispielsweise der Glycidyläther/Glycidylester der Salicylsäure oder p-(Diglycidylamino)-phenylglycidyläther.

Besonders bevorzugt steht $R^3$ für den zweiwertigen Rest eines gegebenenfalls vorverlängerten Diglycidyläthers aus einem zweiwertigen Phenol oder einem zweiwertigen aliphatischen Alkohol.

Als (A) verwendbare Verbindungen mit mindestens einer Gruppe der Formel II, worin $R^2$ für ein Wasserstoffatom steht, lassen sich auch durch eine ähnliche Reaktion zwischen einer Bicyclo[2.2.1]-hept-2-en-6-carbonsäure der Formel III und Glycidylacrylat oder Glycidylmethacrylat erhalten. Solche Verbindungen entsprechen der Formel

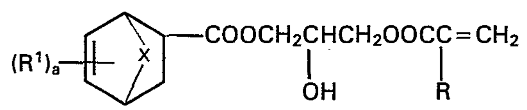

VII

worin R, $R^1$, X und a die oben angegebenen Bedeutungen haben.

Methoden zur Ringöffnung von 1.2-Epoxidgruppen mit Carbonsäuren sind wohlbekannt.

Als Komponente (A) geeignete Verbindungen mit mindestens einer Gruppe der Formel II, worin $R^2$ für ein Wasserstoffatom steht, lassen sich auch durch Veresterung einer Verbindung mit zwei oder mehr alkoholischen oder phenolischen Hydroxylgruppen mit Acryloyl- oder Methacryloylchlorid und mit einem Bicyclo[2.2.1]hept-2-en-6-carbonsäurechlorid der Formel

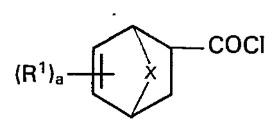

VIII

worin $R^1$, X und a die oben angegebenen Bedeutungen haben, erhalten.

Demnach kann man dabei Ester der Formel

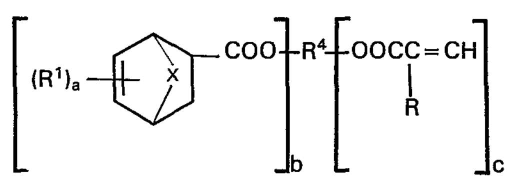

IX

worin R, $R^1$, X, a, b und c die oben angegebenen Bedeutungen haben und $R^4$ den Rest eines Alkohols mit mindestens (b + c) alkoholischen Hydroxylgruppen bzw. eines Phenols mit mindestens (b + c) phenolischen Hydroxylgruppen nach Wegnahme von (b + c) alkoholischen bzw. phenolischen Hydroxylgruppen darstellt, einsetzen.

$R^4$ steht vorzugsweise für einen aliphatischen Rest, der sich wiederholende Einheiten der Formel $-O(CH_2)_f-$ oder $-CO(CH_2)_5O-$ (wobei f 2, 3 oder 4 ist) oder sich wiederholende Einheiten der Formel $-CH_2CH(OH)-$ enthält. So kann $R^4$ beispielsweise für den Rest eines Polyoxyäthylenglykols oder Polyoxypropylenglykols mit einem Durchschnittsmolekulargewicht von 250 bis 5000 oder den Rest eines Polyvinylalkohols mit einem Durchschnittsmolekulargewicht von 500 bis 9000 stehen.

Es versteht sich, dass sich dabei in ähnlicher Weise Nebenprodukte der Formel

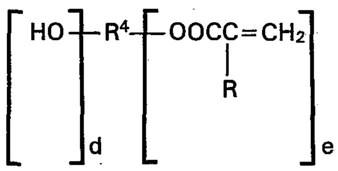

X

und Nebenprodukte der Formel

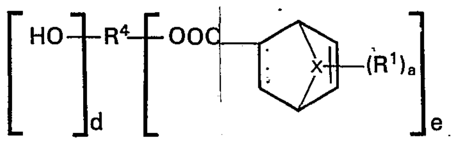

XI

worin R, $R^1$, $R^4$, X, a, d und e die oben angegebenen Bedeutungen haben, bilden werden. Im allgemeinen wird jedoch das erfindungsgemässe Verfahren dadurch nicht gestört.

Ebenfalls zur Verwendung als Komponente (A) geeignete, denen der Formel IX ähnliche Ester entsprechen der Formel

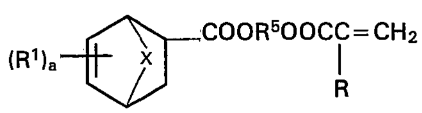

XII

worin R, $R^1$, X und a die oben angegebenen Bedeutungen haben und $R^5$ eine Alkylengruppe mit 2 bis 6 Kohlenstoffatomen bedeutet; diese sind durch Veresterung eines Hydroxyalkyl(meth)acrylats der Formel

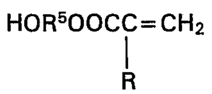

XIII

wie 2-Hydroxyäthylacrylat oder 2-Hydroxypropylmethacrylat mit einem Bicyclo[2.2.1]hept-2-en-6-carbonsäurechlorid der Formel VIII erhältlich.

Als (A) verwendbar Amide der Formel

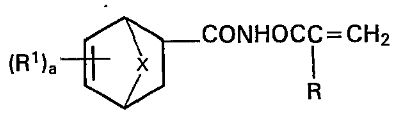

XIV

worin R, $R^1$, X und a die oben angegebenen Bedeutungen haben, lassen sich durch Behandlung von (Meth)acrylamid mit einem Bicyclo[2.2.1]hept-2-en-6-carbonsäurechlorid der Formel VIII oder durch Umsetzung mit (Meth)acryloylchlorid und einem Bicyclo[2.2.1]hept-2-en-6-carboxamid der Formel

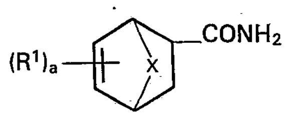

XV

worin $R^1$, X und a die oben angegebenen Bedeutungen haben, erhalten.

In ähnlicher Weise liefert die Reaktion eines Bicyclo[2.2.1]hept-2-en-6-carbonsäurechlorids der Formel VIII mit einem N-Hydroxymethyl-(meth)-acrylamid zur Verwendung als (A) geeignete Amide der Formel

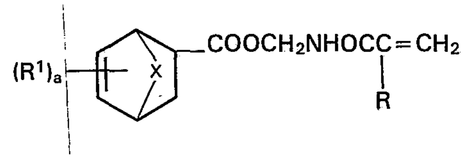

XVI

worin R, $R^1$, X und a die oben angegebenen Bedeutungen haben.

Weitere eine Gruppe der Formel II, worin $R^2$ für ein Wasserstoffatom steht, enthaltende und zur Verwendung als (A) geeignete Ester sind durch Veresterung eines Bicyclo[2.2.1]hept-2-en-6-ols oder eines 6-Hydroxymethyl-bicyclo[2.2.1]hept-2-ens mit (Meth)acryloylchlorid erhältlich; diese entsprechen der Formel

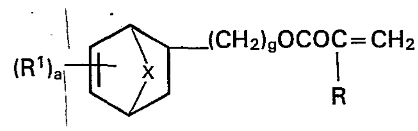

XVII

worin R, $R^1$, X und a die oben angegebenen Bedeutungen haben und g null oder 1 ist.

Ebenfalls kommen Amide in Betracht, die durch Reaktion eines Polyamins, gleichzeitig oder in beliebiger Reihenfolge, mit (Meth)acryloylchlorid und mit einem Bicyclo[2.2.1]hept-2-en-6-carbonsäurechlorid der Formel VIII hergestellt werden, d.h. Amide der Formel

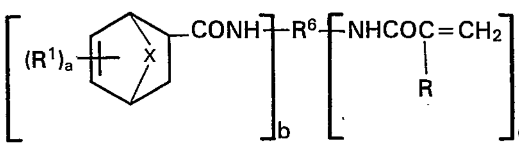

XVIII

worin R, $R^1$, X, a, b und c die oben angegebenen Bedeutungen haben und $R^6$ den Rest einer Verbindung mit mindestens (b + c) primären Aminogruppen nach Wegnahme von (b + c) solchen Aminogruppen darstellt.

Dabei werden sich auf die gleiche Weise Nebenprodukte der Formel

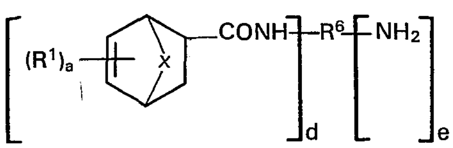

XIX

oder

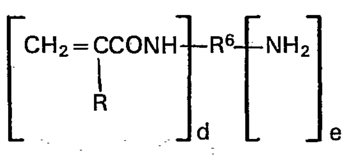

XX

worin R, $R^1$, $R^6$, X, a, d und e die oben angegebenen Bedeutungen haben, bilden, doch stören diese im allgemeinen nicht.

Vorzugsweise bedeutet $R^6$ den Rest eines Alkylendiamins mit 2 bis 8 Kohlenstoffatomen, eines

Phenylendiamins oder eines Bis-(aminophenyl)-methans nach Wegnahme beider primärer Aminogruppen.

Bei einem weiteren Weg handelt es sich um die Umsetzung von Alkoholen der Formel

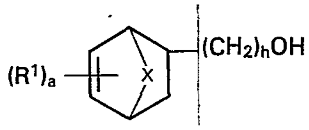
XXI

worin h 0, 1 oder 2 ist und $R^1$, X und a die oben angegebenen Bedeutungen haben, mit einem Disäurechlorid der Formel $ClCOR^7COCl$, worin $R^7$ eine Kohlenstoff-Kohlenstoffbindung oder eine zweiwertige Gruppe mit 1 bis 10 Kohlenstoffatomen bedeutet, und mit einem Hydroxyalkyl-(meth)-acrylat der Formel XIII.

Damit kommen Ester der Formel

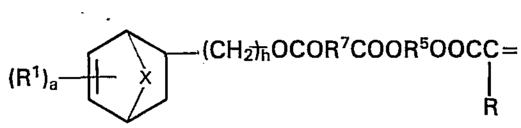
XXII

worin R, $R^1$, $R^5$, $R^7$, X, a und h die oben angegebenen Bedeutungen haben, in Betracht.

Nebenprodukte der Formel

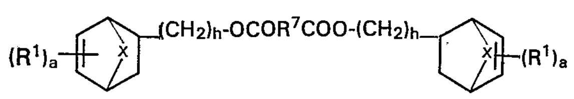
XXIII

und der Formel

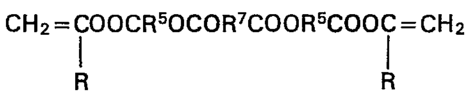
XXIV

worin R, $R^1$, $R^5$, $R^7$, X, a und h die oben angegebenen Bedeutungen haben, bilden sich ebenfalls, doch stören sie im allgemeinen nicht.

Eine Gruppe der Formel II, worin $R^2$ für ein Wasserstoffatom steht, enthaltende und zur Verwendung als (A) geeignete Verbindungen lassen sich auch durch Umsetzung eines 6-Hydroxymethylbicyclo[2.2.1]hept-2-ens mit Glycidyl-(meth)acrylat unter ringöffnenden Bedingungen erhalten. Die so entstehenden 3-(Meth)acryloyloxy-2-hydroxypropoxymethylderivate entsprechen der Formel

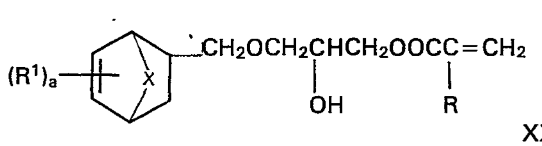
XXV

worin R, $R^1$, X und a die oben angegebenen Bedeutungen haben.

Ferner kann man Carbonate der Formel

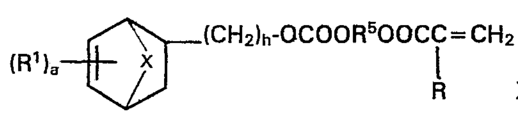
XXVI

worin R, $R^1$, $R^5$, X, a und h die oben angegebenen Bedeutungen haben, verwenden, die durch Reaktion eines Alkohols der Formel XXI und eines Hydroxyalkyl-(meth)acrylats der Formel XIII mit überschüssigem Phosgen erhältlich sind. Dabei können sich auch als Nebenprodukte die Carbonate der Formel

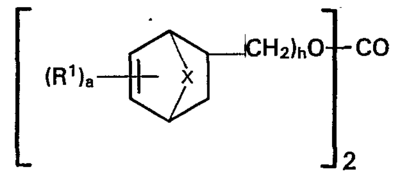
XXVII

und der Formel

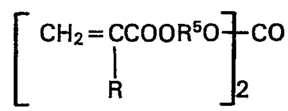
XXVIII

worin R, $R^1$, $R^5$, X a und h die oben angegebenen Bedeutungen haben, bilden, doch stören sie im allgemeinen nicht das erfindungsgemässe Verfahren.

Weitere zur Verwendung als (A) geeignete Verbindungen sind die Diurethane der Formel

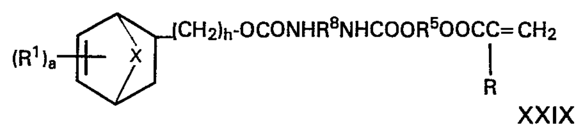
XXIX

worin R, $R^1$, $R^5$, X, a und h die oben angegebenen Bedeutungen haben und $R^8$ den Rest eines organischen Diisocyanats nach Wegnahme der zwei Isocyanatgruppen und vorzugsweise eine Arylen- oder Aralkylengruppe mit 6 bis 15 Kohlenstoffatomen darstellt; diese sind durch Umsetzung eines Diisocyanats mit einem Alkohol der Formel XXI und einem Hydroxyalkylacrylat der Formel XIII erhältlich.

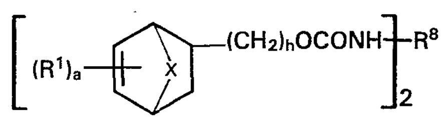
XXX

und der Formel

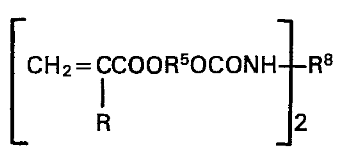
XXXI

worin R, $R^1$, $R^5$, $R^8$, X, a und h die oben angegebenen Bedeutungen haben, können sich dabei als Nebenprodukte bilden, doch stören sie die Durchführung des erfindungsgemässen Verfahrens im allgemeinen nicht.

Weitere Wege zu mindestens eine Gruppe der Formel II, worin $R^2$ für ein Wasserstoffatom steht, enthaltenden und zur Verwendung als (A) geeigneten Verbindungen führen über ein Bicyclo[2.2.1]hept--2-en-6-al als Zwischenprodukt.

So kann man einen solchen Aldehyd durch Reaktion mit Formaldehyd in ein 6,6-Bis-(hydroxymethyl)-bicyclo[2.2.1]hept-2-en umwandeln und dieses Diol durch Veresterung mit zwei Mol (Meth)-acryloylchlorid direkt in ein (Meth)acrylat der Formel

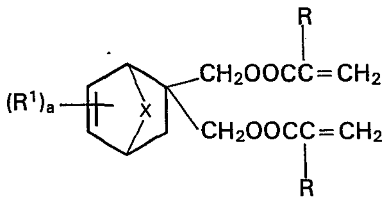

XXXII

worin R, $R^1$, X und a die oben angegebenen Bedeutungen haben, oder in einen Diglycidyläther überführen, den man dann mit (Meth)acrylsäure zu einem Diester der Formel

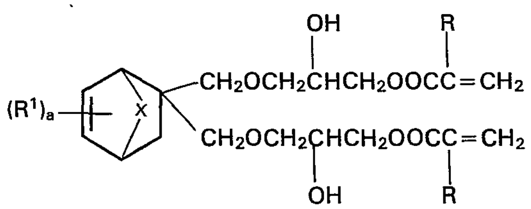

XXXIII

worin R, $R^1$, X und a die oben angegebenen Bedeutungen haben, reagieren lassen kann.

Auch kann man ein Bicyclo[2.2.1]hept-2-en-6-al in beliebiger Reihenfolge mit einem mindestens dreiwertigen Alkohol unter Acetalbildung zur Reaktion bringen und mindestens eine verbleibende alkoholische Gruppe mit (Meth)acryloylchlorid zu Esteracetalen der Formel

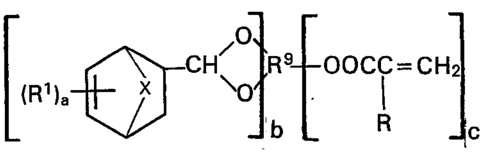

XXXIV

verestern, worin R, $R^1$, X, a, b und c die oben angegebenen Bedeutungen haben und $R^9$ den Rest einer mindestens (2b+c) alkoholische Hydroxylgruppen enthaltenden Verbindung nach Wegnahme von (2b+c) solchen Hydroxylgruppen, wie einen Polyvinylalkohol vom Durchschnittsmolekulargewicht 500 bis 9000, darstellt.

Es versteht sich, dass sich dabei Gruppen der Formel I aber keine der Formel II oder umgekehrt enthaltende Nebenprodukte bilden können, das heisst Verbindungen der Formel

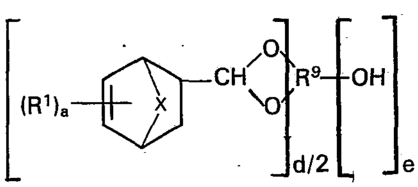

XXXV

oder

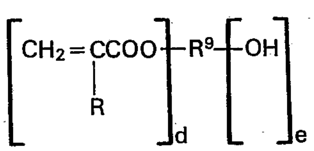

XXXVI

worin R, $R^1$, $R^9$, X, a, d und e die oben angegebenen Bedeutungen haben, doch stören solche Nebenprodukte das erfindungsgemässe Verfahren im allgemeinen nicht.

Zur Verwendung als (A) geeignete phosphorhaltige Verbindungen entsprechen der Formel

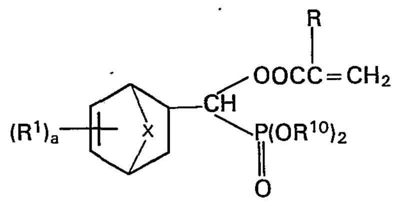

XXXVII

und sind durch Umsetzung eines Bicyclo[2.2.1]hept-2-en-6-als mit einem Phosphonat der Formel $(R^{10}O)_2PHO$ unter Bildung des Alkohols der Formel

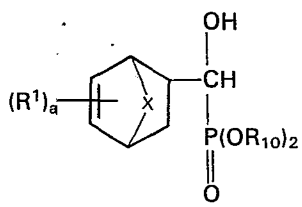

XXXVIII

und nachfolgende Veresterung mit (Meth)acryloylchlorid erhältlich, wobei R, $R^1$, X und a die oben angegebenen Bedeutungen haben und $R^{10}$ jeweils eine gegebenenfalls durch ein oder zwei Äthersauerstoffatome unterbrochene Alkylgruppe mit 1 bis 6 Kohlensstoffatomen, eine Alkenylgruppe mit 2 bis 6 Kohlenstoffatomen, eine Cycloalkylgruppe mit 5 bis 7 Kohlenstoffatomen, eine Arylgruppe mit 6 bis 9 Kohlenstoffatomen oder eine Aralkylgruppe mit 7 bis 10 Kohlenstoffatomen darstellt.

Noch weitere, bei der Herstellung von mindestens eine Gruppe der Formel II, worin $R^2$ von einem Wasserstoffatom verschieden ist, enthaltenden und zur Verwendung als (A) geeigneten Verbindungen nützliche Zwischenprodukte sind Bicyclo[2.2.1]hept-2-en-5,6-dicarbonsäureanhydride. Dies kann man mit einem Hydroxyalkyl-(meth)acrylat der Formel XIII unter Bildung eines sauren Esters der Formel

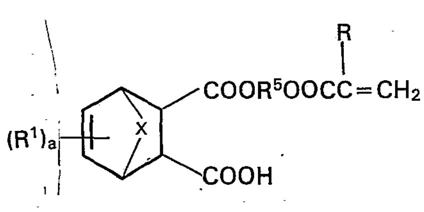

XXXIX

worin R, $R^1$, $R^5$, X und a die oben angegebenen Bedeutungen haben, reagieren lassen.

Gewünschtenfalls kann man solche sauren Ester mit aliphatischen, gegebenenfalls ein Äthersauerstoffatom in der Kette enthaltenden Alkoholen mit bis zu 7 Kohlenstoffatomen, z.B. 2-n-Butoxyäthanol, weiter verestern.

Auch kann man ein solches Anhydrid in die 5,6-Dicarbonsäure umwandeln, die sich auf ähnliche Weise mit einem Hydroxyalkyl-(meth)acrylat der Formel XIII (2 Mol) zu einem Diester der allgemeinen Formel

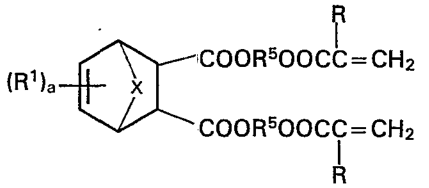

XL

worin R, $R^1$, $R^5$, X und a die oben angegebenen Bedeutungen haben, verestern lässt.

Bei weiteren Wegen zu als (A) verwendbaren Verbindungen aus Bicyclo[2.2.1]hept-2-en-5,6-dicarbonsäureanhydriden handelt es sich um die Bildung eines Halbesters mit einem einwertigen Alkohol der Formel $R^{11}OH$ und anschliessend entweder um die Umwandlung des Halbesters in ein Säurechlorid (durch Umsetzung mit z.B. Thionylchlorid) und danach Veresterung mit einem Hydroxyalkyl-(meth)-acrylat der Formel XIII oder eine Behandlung des Halbesters mit Glycidyl-(meth)acrylat zur Bildung des 3-(Meth)acryloyl-2-hydroxypropylesters.

Damit kommen Verbindung der Formel

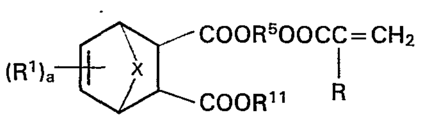

XLI

oder die Formel

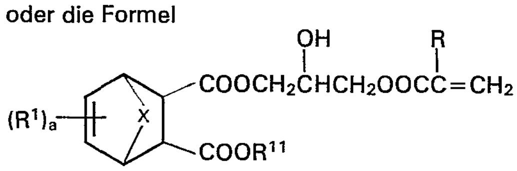

XLII

worin R, $R^1$, $R^5$, X und a die oben angegebenen Bedeutungen haben und $R^{11}$ eine gegebenenfalls in der Kette durch ein Äthersauerstoffatom unterbrochene Alkylgruppe mit 1 bis 8 Kohlenstoffatomen darstellt, in Betracht.

Noch weitere als (A) verwendbare Verbindungen sind substituierte Imide der Formel

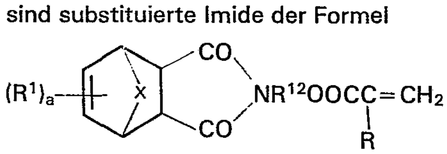

XLIII

worin R, $R^1$, X und a die oben angegebenen Bedeutungen haben und $R^{12}$ eine Alkylengruppe mit 1 bis 6 Kohlenstoffatomen darstellt.

Solche Verbindungen sind aus Bicyclo[2.2.1]hept-2-en-5,6-dicarbonsäureanhydriden entweder durch Umwandlung in das Imid und anschliessende Reaktion mit Formaldehyd zum N-Methylolimid oder durch Umsetzung mit einem Aminoalkohol der Formel $NH_2R^{12}OH$, jeweils mit nachfolgender Veresterung mit (Meth)acryloylchlorid, erhältlich.

Als (A) sind solche Verbindungen besonders bevorzugt, die eine Gruppe der Formel II enthalten, worin a 1 oder null ist und X eine unsubstituierte Methylenbrücke, d.h. $-CH_2-$, darstellt.

Die zur Herstellung von Verbindungen der Formeln IV, VII, IX, XII, XIV, XVI-XVIII, XXII, XXV, XXVI, XXIX, XXXII bis XXXIV, XXXVIII und XXXIX bis XLIII erforderlichen Zwischenprodukte sind im allgemeinen wohlbekannt (s. z.B. britische Patentschrift Nr. 935 756); diese werden üblicherweise durch eine Diels-Alder Reaktion zwischen gegebenenfalls durch bis zu 4 Methylgruppen oder eine Allylgruppe substituiertem Cyclopentadien oder Furan und einem geeigneten Dienophil wie Acrylsäure, Acrolein oder Maleinsäureanhydrid hergestellt. Einige der sich von Cyclopentadien ableitenden Verbindungen sind im Handel erhältlich.

Spezielle Beispiele für zur Verwendung als (A) geeignete Substanzen sind Bicyclo[2.2.1]hept-2-en-6-carbonsäure-3-(methacryloyloxy)-2-hydroxypropylester, Methyl-5-carboxybicyclo[2.2.1]hept-2-en-6-carbonsäure-2-(acryloyloxy)-äthylester und die entsprechende Methacryloyloxyverbindung, 5-Carboxy-7-oxabicyclo[2.2.1]hept-2-en-6-carbonsäure-2-(acryloyloxy)-äthyelester, Allyl-5-carboxybicyclo[2.2.1]hept-2-en-6-carbonsäure-2-(acryloyloxy)-propylester, Methyl-5-carbomethoxybicyclo[2.2.1]hept-2-en-6-carbonsäure-3-(methacryloyloxy)-2-hydroxypropylester, 5-Carboxy-7-oxabicyclo[2.2.1]hept-2-en-6-carbonsäure-2-(methacryloyloxy)-äthylester und N-(2-Methacryloyloxyäthyl)-methylbicyclo[2.2.1]hept-2-en-5,6-dicarboximid.

Die zweite dieser Verbindungen wird aus Methylbicyclo[2.2.1]hept-2-en-5,6-dicarbonsäureanhydrid, das man seinerseits durch eine Diels-Alder Reaktion zwischen Methylcyclopentadien und Maleinsäureanhydrid erhält, hergestellt. Es versteht sich, dass man der Methylgruppe in Methylcyclopentadien keine bestimmte Stellung zuschreiben kann, da das Handelsprodukt aus Gemischen besteht, die isomerisieren können. Daraus folgt, dass man auch dem Addukt mit Maleinsäureanhydrid oder natürlich den Derivaten des so hergestellten Bicyclo[2.2.1]hept-2-en-5,6-dicarbonsäureanhydrids keine speziellen Stellungen zuteilen kann. Das gleiche gilt für beim erfindungsgemässen Verfahren verwendete Homologe, die bis zu vier Methylgruppen im Bicyclo[2.2.1]-hept-2-enkern enthalten. Ebenso lässt sich der Allylgruppe im Allylcyclopentadien, aus dem mindestens eine Gruppe der Formel II, worin $(R^1)_a$ eine Allylgruppe bedeutet, enthaltende Verbindungen herstellbar sind, keine bestimmte Stellung zuschreiben.

Zur Photopolymerisation über Gruppen der Formel I ist es bei weitem vorzuziehen, dass die flüssige Zusammensetzung einen zugesetzten Photoinitiator enthält, d.h. einen Katalysator, der bei Bestrahlung einen angeregten Zustand ergibt, der zur Bildung freier Reste führt, die dann die Polymerisation von (A) einleiten. Beispiele für geeignete Photoinitiatoren sind organische Peroxyde und Hydroperoxyde, α-halogensubstituierte Acetophenone wie Trichlormethyl-4'tert.-butylphenylketon, α-hydroxy-α-alkyl-substituierte Acetophenone wie 2-Hydroxy-2-methyl-1-phenylpropanon-1, Benzoin und dessen Alkyläther (z.B. der n-Butyläther), α-Methylbenzoin, α,α-Dialkyloxy-α-benzoylessigsäurealkylester, Benzophenone wie Benzophenon selbst und 4,4'-Bis-(dimethylamino)-benzophenone, O-Alkoxycarbonylderivate eines Oxims des Benzils oder 1-Phenylpropan-1,2-dions, wie Benzyl-(O-äthoxycarbonyl)-α-monoxim und 1-Phenylpropan-1,2-dion-2-(O-äthoxycarbonyl)-oxim, Benzylketale, z.B. dessen Dimethylketal, substituierte Thioxanthone, z.B. 2-Chlorthioxanthon, Anthrachinone, Ester der Phenylglyoxylsäure, 2-Benzoyl-2-phenyl-1,3-dioxolane und 4-Benzoyl-4-phenyl-1,3-dioxolane sowie Photoredoxsysteme, die aus einem Gemisch aus einem Phenothiazinfarbstoff (z.B. Methylenblau) oder

einem Chinoxalin [z.B. einem Metallsalz der 2-(m- oder p-Methoxyphenyl)-chinoxalin-6'- oder -7'-sulfonsäure] mit einem Elektronendonator wie Benzolsulfinsäure oder einer anderen Sulfinsäure oder einem Salz davon, z.B. dem Natriumsalz, oder einem Arsin, einem Phosphin oder Thioharnstoff bestehen.

Geeignete Photoinitiatoren lassen sich leicht durch Serienversuche auffinden. Im allgemeinen werden 0,15 bis 10 Gew.-% und vorzugsweise 2,5 bis 5 Gew.-% Photoinitiator zugegeben, bezogen auf das Gesamtgewicht von (A), und gegebenenfalls einer weiteren Verbindung (B) mit mindestens einer Gruppe der Formel I aber keiner der Formel II. [Zur Verwendung als Verbindung (B) geeignete Substanzen sind weiter unten beschrieben.]

Bezugnahmen auf Photovernetzung über Gruppen der Formel II sind in dieser Patentschrift nicht so auszulegen, als dass sie die Möglichkeit ausschliessen würden, dass ein geringes Ausmass von Photodimerisierung über Gruppen der Formel II in der Stufe (1) stattfinden könnte; es wird jedoch angenommen, dass die Photopolymerisation in Stufe (1) weit überwiegend nur Gruppen der Formel I betrifft.

Wie oben erwähnt wird die Zusammensetzung in flüssiger Form auf einen Träger aufgebracht. Zweckmässig liegt ihre Viskosität im Bereich 0,1 bis 0,4 Pa.s. Um das Erfordernis einer flüssigen Zusammensetzung zu erfüllen , kann es nötig sein, um dies ohne Anwendung flüchtiger organischer Lösungsmittel zu erreichen, eine weitere Verbindung mitzuverwenden, die flüssig ist und unter den Bedingungen der Stufe (1) unter Bildung eines Feststoffs photopolymerisiert. Hierfür ist es zweckmässig, eine photopolymerisierbare Verbindung (B) mit mindestens einer Gruppe der Formel I aber keiner der Formel II im Molekül zu verwenden. Die Verbindung (B) kann beispielsweise ein gegebenenfalls substituierter Alkyl- oder Hydroxyalkylester der Acrylsäure oder Methacrylsäure sein, wobei solche Ester typischerweise insgesamt bis 15 Kohlenstoffatome aufweisen, wie Methylmethacrylat, Äthylmethacrylat, n-Butylacrylat und 2-Hydroxyäthylacrylat. Ebenfalls kommen hierfür die 3-Alkoxy-2-hydroxypropyl-, 3-Alkenoxy-2--hydroxypropyl- und 3-Aryloxy-2-hydroxypropylester der Acrylsäure oder Methacrylsäure in Betracht, wobei diese typischerweise insgesamt bis 15 Kohlenstoffatome enthalten.

Weitere Arten Verbindung (B) können der flüssigen Zusammensetzung zugegeben werden, um dem photopolymerisierten, photovernetzten Produkt gewisse erwünschte Eigenschaften zu verleihen. So kann die Verbindung (B) auch mindestens ein Chlor-, Brom- oder Phosphoratom enthalten, um flammhemmende Eigenschaften zu verleihen. Beispiele dafür sind Acrylsäure- oder Methacrylsäureaddukte mit einem brom- oder chlorsubstituierten Arylglycidyläther wie Dibrom-p-kresyl-glycidyläther, z.B. 3--(Methacryloyloxy)-2-hydroxypropyl-X,Y-dibrom-p--kresyläther.

Es hat sich als vorteilhaft erwiesen, in die flüssige Zusammensetzung ferner als Verbindung (B) eine solche einzuarbeiten, die im Molekül sowohl mindestens eine Gruppe der Formel I als auch mindestens eine Gruppe der Formel

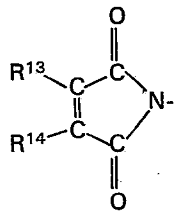

XLIV

worin $R^{13}$ und $R^{14}$, die gleich oder verschieden sein können, je eine Alkylgruppe mit 1 bis 4 Kohlenstoffatomen oder zusammen eine gegebenenfalls durch eine Methylgruppe substituierte Trimethylen- oder Tetramethylengruppe bedeuten, enthält. Die Gegenwart einer solchen Verbindung führt im allgemeinen zu verbesserter Haftung des photovernetzten Produkts am Substrat.

Sowohl mindestens eine Gruppe der Formel XLIV als auch mindestens eine Gruppe der Formel I im Molekül enthaltende Verbindungen sind in der britischen Patentschrift Nr. 1 544 840 beschrieben. Üblicherweise enthält die Verbindung pro Durchschnittsmolekül bis vier Gruppen der Formel XLKIV und bis vier Gruppen der Formel I. Vorzugsweise besitzt sie ein Molekulargewicht von höchstens 10 000, und vorzugsweise sind die Gruppe oder Gruppen der Formel XLIII sowie die Gruppe oder Gruppen der Formel I jeweils direkt an ein bzw. mehrere Kohlenstoff-, Sauerstoff- oder Stickstoffatom(e) gebunden. Besonders bevorzugt sind solche Verbindungen, worin $R^{13}$ und $R^{14}$ in der Formel XLIV beide für eine Methylgruppe stehen.

Solche Verbindungen können der Formel

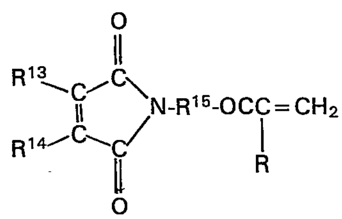

XLV

entsprechen, worin R, $R^{13}$ und $R^{14}$ die oben angegebenen Bedeutungen haben und $R^{15}$ eine Iminogruppe, eine gegebenenfalls an einem ihrer Kohlenstoffatome durch eine Hydroxylgruppe substituierte Alkylenoxygruppe mit 1 bis 10 Kohlenstoffatomen, eine Cycloalkylenoxygruppe mit 5 bis 7 Kohlenstoffatomen oder eine Alkylencarbonamidogruppe mit 2 bis 10 Kohlenstoffatomen darstellt.

$R^{15}$ kann beispielsweise für eine Alkylencarbonamidogruppe der Formel $-(CH_2)_5CONH-$, eine Alkylenoxygruppe der Formel $-(CH_2)_6O-$, eine Cyclohexylenoxygruppe oder eine Methylcyclohexylenoxygruppe stehen; vorzugsweise bedeutet es eine Gruppe der Formel $-(Ch_2)_jO-$, $-CH_2CH(CH_3)O-$ oder $-CH_2CH(OH)CH_2O-$, wobei j 1, 2 oder 3 ist.

Verbindungen der Formel XLV, worin $R^{15}$ eine Iminogruppe bedeutet, sind aus einem 2,3-disubstituierten N-Aminomaleinimid durch Behandlung mit Acryloylchlorid oder Methacryloylchlorid erhältlich.

Verbindungen der Formel XLV, worin $R^{15}$ eine Alkenoxygruppe mit mindestens zwei Kohlenstoffatomen bedeutet, kann man durch Umsetzung eines

2,3-disubstituierten Maleinsäureanhydrids mit einem Aminoalkohol und nachfolgende Behandlung mit Acryloylchlorid oder Methacryloylchlorid erhalten, während solche, worin $R^{15}$ $-CH_2O-$ bedeutet, durch Überführung eines 2,3-disubstituierten Maleinimids in sein N-Hydroxymethylderivat mittels Formaldehyd und nachfolgende Behandlung mit (Meth)acryloylchlorid erhältlich sind. Verbindungen, worin $R^{15}$ für $-CH_2CH(OH)CH_2O-$ steht, kann man durch Umsetzung eines 2,3-disubstituierten N-Glycidylmaleinimids mit Acrylsäure oder Methacrylsäure erhalten. Verbindungen der Formel XLV, worin $R^{15}$ eine Cycloalkenoxygruppe bedeutet, sind durch Reaktion eines 2,3-disubstituierten Maleinsäureanhydrids mit einem cycloaliphatischen Aminoalkohol und nachfolgende Behandlung mit Acryloylchlorid oder Methacryloylchlorid herstellbar.

Verbindungen der Formel XLV, worin $R^{15}$ eine Alkylencarbonamidogruppe bedeutet, können durch Umsetzung eines 2,3-disubstituierten Maleinsäureanhydrids mit einer aminosubstituierten aliphatischen Carbonsäure unter Bildung des 2,3-disubstituierten N-Carboxyalkylenmaleinimids, Überführung in das Säurechlorid und Umsetzung mit Acrylamid oder Methacrylamid erhalten werden.

Spezielle Beispiele für solche Substanzen sind N-[2-(Acryloyloxy)-äthyl]-2,3-dimethylmaleinimid, N-[3-(Acryloyloxy)-2-hydroxypropyl]-2,3-dimethylmaleinimid, N-[3-(Acryloyloxy)-propyl]-2,3-dimethylmaleinimid und die entsprechenden Methacryloylhomologen.

Wie unten erläutert, kann es wünschenswert sein, Epoxidgruppen in die Zusammensetzung einzuführen, denn nach der Photovernetzung des Produkts lässt sich weitere Vernetzung durch Heisshärtung über die Epoxidgruppen erzielen. Es kann daher vorteilhaft sein, in der flüssigen Zusammensetzung eine photopolymerisierbare Verbindung mitzuverwenden, die im selben Molekül sowohl eine Gruppe der Formel I als auch nur eine 1,2-Epoxidgruppe aufweist, wie Glycidylacrylat oder Glycidylmethacrylat. Ferner kann man der flüssigen Zusammensetzung vor der Photopolymerisation ein Epoxidharz (d.h. eine mehr als eine Epoxidgruppe enthaltende Verbindung) zufügen.

Gewünschtenfalls kann die flüssige Zusammensetzung ferner eine photopolymerisierbare Verbindung (C) mit mindestens einer Gruppe der Formel II aber keiner der Formel I enthalten, z.B. Bicyclo-[2.2.1]hept-2-en.

Die flüssige Zusammensetzung lässt sich nach herkömmlichen Methoden wie Sprühbeschichtung, Aufschleudern, Walzenauftrag, Kaskadenbeschichtung und insbesondere Vorhangbeschichtung auf geeignete Träger aufbringen. Typischerweise wird der Träger so beschichtet, dass die Schicht der Zusammensetzung 1 bis 250 $\mu m$ dick ist. Der Träger kann beispielsweise aus Kupfer, Aluminium oder einem anderen Metall, aus Papier, Kunstharz oder Glas bestehen.

Sowohl bei der Photopolymerisationsstufe als auch der nachfolgenden Photovernetzungsstufe des erfindungsgemässen Verfahrens verwendet man bevorzugt aktinische Strahlung einer Wellenlänge von 200-600 nm. Als aktinische Strahlungsquellen eignen sich unter anderem Kohlelichtbögen, Quecksilberdampflichtbögen, Leuchtröhren mit ultraviolettes Licht ausstrahlenden Leuchtstoffen, Argon- und Xenonglimmlampen, Wolframlampen und photographische Flutlampen. Darunter sind Quecksilberdampflichtbögen, insbesondere Höhensonnen, fluoreszierende Höhensonnen und Metallhalogenidlampen am besten geeignet. Die zur Belichtung der photopolymerisierbaren Zusammensetzung und der noch photovernetzbaren Zusammensetzung erforderlichen Zeiten hängen von verschiedenen Faktoren ab, unter anderem beisielsweise den individuellen verwendeten Verbindungen, den Art der Lichtquelle und deren Abstand von der bestrahlten Zusammensetzung. Der mit Photopolymerisationsmethoden vertraute Fachmann kann die geeigneten Zeiten leicht bestimmen; im allgemeinen ist die in der zweiten Stufe (der Photovernetzungsstufe) benötigte Lichtenergiemenge 15- bis 100mal, typischerweise 25- bis 60mal, grösser als in der ersten Stufe. Zum Beispiel wird die Zusammensetzung zunächst im Abstand von 10-25 cm von einer Strahlungsquelle 2-15 Sekunden lang bestrahlt; in der zweiten Stufe erfolgt die Bestrahlung 10-20 Minuten lang im Abstand von 15-30 cm.

Die zur Verwendung als Verbindung (A) bevorzugten Substanzen enthalten nur eine Gruppe der Formel I pro Molekül, doch wurden befriedigende Ergebnisse mit mehr als eine solche Gruppe enthaltenden Substanzen erzielt. Bei Verwendung von Diacrylaten, Dimethacrylaten und anderen mehr als eine Gruppe der Formel I enthaltenden Substanzen soll die Belichtung mit aktinischer Strahlung in Stufe (1) begrenzt sein, damit eventuelle Vernetzung über Gruppen der Formel I in dieser Stufe nicht so weit fortschreitet, dass die Erzeugung einer Abbildung in Stufe (3) erheblich gehemmt wird.

Zum Entwickeln der Abbildung geeignete Lösungsmittel lassen sich leicht durch Serienversuche auffinden; dies sind unter anderem Cyclohexanon, Trimethylcyclohexanon, 2-Äthoxyäthanol, 1,1,1-Trichloräthan und deren Gemische. Es kann notwendig sein, die Wirkung des Lösungsmittels durch Rühren oder leichtes Bürsten zu unterstützen. Besitzt der Träger eine Schicht eines geeigneten elektrisch leitenden Metalls, üblicherweise Kupfer oder Silber, in direkter Berührung mit der photopolymerisierten Zusammensetzung, so kann das unvernetzte Polymer gegebenenfalls entfernt werden, um das Metall freizulegen. So freigelegtes Metall kann dann an den Nicht-bildstellen mittels Ätzflüssigkeiten wie Ferrichlorid- oder Ammoniumpersulfatlösungen weggeätzt werden, um eine gedruckte Schaltung zu bilden.

Gewünschtenfalls kann man in der flüssigen Zusammensetzung eine Verbindung (B) mitverwenden, welche ferner mindestens eine freie Sulfon- oder Phosphonsäuregruppe oder insbesondere mindestens eine freie Carboxylgruppe aufweist; zweckmässig ist dies Acrylsäure oder Methacrylsäure oder ein Anlagerungsprodukt eines Hydroxyalkylacrylats oder -methacrylats der Formel XIII mit Trimellithsäureanhydrid, d.h. ein Addukt der Formel

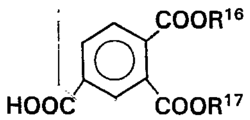

XLVI

worin $R^{16}$ entweder ein Wasserstoffatom bedeutet, in welchem Fall $R^{17}$ dann eine Gruppe der Formel

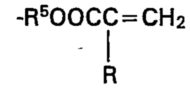

XLVII

darstellt, oder $R^{16}$bedeutet eine Gruppe der Formel XLVII, in welchem Fall $R^{17}$ dann für ein Wasserstoffatom steht, wobei R und $R^5$ die oben angegebenen Bedeutungen haben.

Anstelle von oder zusätzlich zu einer solchen Verbindung (B) kann man eine carboxylhaltige Verbindung (A) der Formel XXXIX einsetzen.

Die Gegenwart freier Sulfon-, Phosphon- oder Carbonsäuregruppen im photopolymerisierten Polymer hat zur Folge, dass man zur Entwicklung der Abbildung eine wässrige Lösung einer Base, wie verdünnte Natronlauge, Natriumcarbonat-, Dinatriumhydrogenorthophosphat- oder Ammoniaklösungen verwenden kann, wodurch die Anwendung organischer Lösungsmittel in dieser Stufe vermieden wird.

Andererseits kann man in der flüssigen Zusammensetzung auch eine Verbindung (B) mitverwenden, welche ferner mindestens eine primäre, sekundäre oder tertiäre Aminogruppe trägt. Als Folge der Gegenwart freier Aminogruppen im photopolymerisierten Polymer kann man wässrige Säurelösungen wie verdünnte Säurelösungen, insbesondere Mineralsäuren zur Entwicklung der Abbildung einsetzen. Beispiele für solche Verbindungen (B) sind Alkylester der Acryl- oder Methacrylsäure, deren Alkylgruppen durch eine sekundäre oder tertiäre Aminogruppe substituiert sind, wie 2-(Dimethylamino)-äthyl-methacrylat.

Wie bereits angegeben kann die Zusammensetzung nach der Photopolymerisation und Photovernetzung ein Epoxidharz enthalten, wobei sie dann ferner einen latenten Heisshärter für das Epoxidharz enthalten kann, so dass die Zusammensetzung erhitzt werden und zusätzliche Vernetzung eintreten kann, um die Beständigkeit des photovernetzten Produkts gegen Lösungsmittel und hohe Temperaturen weiter zu steigern. Wie schon angegeben kann das Epoxidharz als solches in die Zusammensetzung eingearbeitet oder an Ort und Stelle durch Photopolymerisation einer zusätzlich nur eine 1,2-Epoxidgruppe im gleichen Molekül enthaltenden Verbindung (B) gebildet werden. Latente Heisshärter für Epoxidharze sind beispielsweise Polycarbonsäureanhydride wie Hexahydrophthalsäureanhydrid, Dicyandiamid, Bortrifluorid- oder Bortrichloridkomplexe von Aminen wie Äthylamin, Trimethylamin und n-Octyldimethylamin, latente Bordifluoridchelate, aromatische Polyamine wie Bis-(p-aminophenyl)-methan und Bis-(p-aminophenyl)-sulfon, aromatische Biguanide wie 2,6-Xylidenbiguanid und Imidazole wie 2-Äthyl-4-methylimidazol und 2-Phenylimidazol. Als latenter Heisshärter kann auch eine an Ort und Stelle durch Photopolymerisation einer Monocarbonsäureverbindung (B) oder einer Verbindung (A) der Formel XXXIX gebildete Polycarbonsäure vorliegen.

Die nachfolgenden Beispiele erläutern die Erfindung. Falls nicht anders angegeben, sind Teile und Prozentangaben Gewichtsteile und Gewichtsprozente. Die in den Beispielen verwendeten Substanzen werden wie folgt hergestellt:

*Bicyclo[2.2.1]hept-2-en-6-carbonsäure-3-(methacryloyloxy)-2-hydroxypropylester*

Man erhitzt 0,13 g 2,6-Di-tert.-butyl-p-kresol und 0,26 g Tetramethylammoniumchlorid enthaltendes Glycidylmethacrylat (43 g) auf 100°C und versetzt im Verlauf von etwa 1 Stunde langsam mit Bicyclo-[2.2.1]hept-2-en-6-carbonsäure (41,8 g), so dass die Temperatur des Gemischs 105°C nicht übersteigt. Bei 100°C wird noch drei Stunden weiter erhitzt, wonach der Epoxidgehalt des Produkts vernachlässigbar ist. Der oben erwähnte Ester ist eine farblose bewegliche Flüssigkeit.

*Methyl-5-carboxybicyclo[2.2.1]hept-2-en-6-carbonsäure-2-(acryloyloxy)-äthylester*

Man erhitzt Methylbicyclo[2.2.1]hept-2-en-5,6-dicarbonsäureanhydrid (40 g) und 2-Hydroxyäthylacrylat (26 g) in 250 ml Toluol 3 Stunden auf 100°C und destilliert dann das Toluol unter vermindertem Druck ab, wobei der oben erwähnte Halbester als viskose Flüssigkeit zurückbleibt.

*Methyl-5-carboxybicyclo[2.2.1]hept-2-en-6-carbonsäure-2-(methacryloyloxy)-äthylester*

Dieser wird auf ähnliche Weise aus 2-Hydroxyäthylmethacrylat hergestellt.

*Methyl-5-carbomethoxybicyclo[2.2.1]hept-2-en-6-carbonsäure-3-(methacryloyloxy)-2-hydroxypropylester*

Man erhitzt ein Gemisch aus Methylbicyclo-[2.2.1]hept-2-en-5,6-dicarbonsäureanhydrid (50 g) und 0,02 g N-Benzyldimethylamin als Katalysator enthaltendem, wasserfreiem Methanol (200 ml) 7 Stunden lang unter Rückfluss und destilliert dann das überschüssige Methanol bei vermindertem Druck ab, wobei Methyl-5-carbomethoxybicyclo[2.2.1]hept-2-en-6-carbonsäure verbleibt (Ausbeute 59 g).

Diesen sauren Ester (50 g) erhitzt man bei 100°C mit Glycidylmethacrylat (40 g) in Gegenwart von 0,27 g Tetramethylammoniumchlorid und 0,13 g 2,6-Di-tert.-butyl-p-kresol, bis der Epoxidgehalt auf einen vernachlässigbaren Wert gefallen ist. Der dabei gebildete gewünschte Ester ist eine viskose Flüssigkeit.

*5-Carboxy-7-oxabicyclo[2.2.1]hept-2-en-6-carbonsäure-2-(acryloyloxy)-äthylester*

Man erhitzt Maleinsäureanhydrid (50 g) und Furan (150 ml) 1 Stunde unter Rückfluss und destilliert dann das überschüssige Furan ab. Der Rückstand wird aus Chloroform umkristallisiert, was 72 g 7-Oxabicyclo[2.2.1]hept-2-en-5,6-dicarbonsäureanhydrid (Schmelzpunkt 110°C, Zers.) liefert. Dieses Anhydrid (40 g) und 30,75 g 2-Hydroxyäthylacrylat erhitzt man in 250 ml Toluol 3 Stunden auf 100°C

und destilliert dann das Toluol bei vermindertem Druck ab, wobei die gewünschte Verbindung als viskose Flüssigkeit zurückbleibt.

*N-(2-Methacryloyloxyäthyl)-methylbicyclo[2.2.1]-hept-2-en-5,6-dicarboximid*

Unter ständigem Rühren gibt man Äthanolamin (91,5 g) allmählich zu auf 120-125°C erhitztem Methylbicyclo[2.2.1]hept-2-en-5,6-dicarbonsäureanhydrid (268,5 g) und erhitzt das Gemisch dann für insgesamt 30 Minuten bei dieser Temperatur. Das Produkt wird auf Raumtemperatur abgekühlt, in Dichlormethan (750 ml) aufgenommen, dann mit 1 n-Natronlauge (150 ml) und Wasser (2mal je 250 ml) gewaschen, über wasserfreiem Magnesiumsulfat getrocknet und dann filtriert. Beim Abdampfen des Lösungsmittels aus dem Filtrat verbleiben 290 g (89% Ausbeute) N-(2-Hydroxyäthyl)-methylbicyclo[2.2.1]hept-2-en-5,6-dicarboximid.

Dieses Imid (222 g) und über Natriumhydroxyd getrocknetes Triäthylamin (111 g) löst man in 470 ml über Natrium getrocknetem, 0,2% 2,6-Di-tert.-butyl-4-methylphenol enthaltendem Diäthyläther. Die Lösung wird auf unter 0°C abgekühlt und tropfenweise mit Methacryloylchlorid (105 g) versetzt, wobei man die Temperatur des Gemischs im Bereich —10°C bis —5°C hält. Nach beendeter Zugabe rührt man weiter und lässt das Gemisch sich auf Raumtemperatur erwärmen. Nach dem Abfiltrieren des ausgefallenen Triäthylaminhydrochlorids wäscht man das Filtrat mit 0,5 n-Salzsäure (65 ml) und Wasser (2mal 65 ml) und trocknet dann über wasserfreiem Magnesiumsulfat. Nach Filtrieren und Abdampfen des Lösungsmittels verbleiben 213 g (73%) des erwünschten N-(Methacryloyloxyäthyl)-imids als goldorangefarbiges Öl.

*5-Carboxy-7-oxabicyclo[2.2.1]hept-2-en-6-carbonsäure-2-(methacryloyloxy)-äthylester*

Man erhitzt ein Gemisch aus 7-Oxabicyclo[2.2.1]-hept-2-en-5,6-dicarbonsäureanhydrid (41,5 g), 2-Hydroxyäthylmethacrylat (32,5 g), Tetramethylammoniumchlorid (0,22 g) und 2,6-Di-tert.-butyl-4-methylphenol (0,15 g) auf 90°C und rührt 10 Stunden bei dieser Temperatur, wobei man den oben genannten Halbester (60 g) erhält.

*N-(2-Methacryloyloxyäthyl)-2,3-dimethylmaleinimid*

Dies wird analog der in Beispiel 17 der britischen Patentschrift Nr. 1 544 840 zur Herstellung des 3-(Methacryloyloxy)-propylhomologen aus N-(2-Hydroxyäthyl)-2,3-dimethylmaleinimid und Methacryloylchlorid hergestellt.

*3-Methacryloyloxy)-2-hydroxypropyl-X,Y-dibrom-p-kresyläther*

Man versetzt 250 g handelsüblichen X,Y-Dibrom-p-kresylglycidyläther (Epoxidgehalt 2,76 val/kg, berechnet 3,11) mit 2,6-Di-tert.-butyl-p-kresol (0,5 g) und 1 g Tetramethylammoniumchlorid und erhitzt die Lösung unter Rühren auf 100°C. Dann gibt man im Verlauf von 1 Stunde Methacrylsäure (59,34 g) langsam mit einer solchen Geschwindigkeit zu, dass die Temperatur des Gemischs 105°C nicht übersteigt. Man erhitzt bei 100°C weiter, bis der Epoxidgehalt auf einen vernachlässigbar kleinen Wert gefallen ist. Das Produkt ist eine klare viskose Flüssigkeit.

*Beispiel 1*

Man bringt eine Zusammensetzung aus 10 Teilen Bicyclo[2.2.1]hept-2-en-6-carbonsäure-3-(methacryloyloxy)-2-hydroxypropylester und 1 Teil Benzyldimethylketal mit einer Viskosität von etwa 0,25 Pa.s durch Wirbelauftrag als ungefähr 20 µm dicke Schicht auf eine Kupferplatte auf. Die Beschichtung wird 10 Sekunden im Abstand von 20 cm mit einer Mitteldruckquecksilberlampe (80 W pro cm) bestrahlt, wobei der Überzug klebfrei wird.

Danach wird er durch ein Negativ hindurch im Abstand von 25 cm 15 Minuten mit einer Mitteldruckquecksilberlampe (30 W pro cm) bestrahlt, worauf man mit Cyclohexanon entwickelt. Man erhält eine gute Reliefabbildung.

*Beispiel 2*

10% Benzyldimethylketal enthaltender Methyl-5-carboxybicyclo[2.2.1]hept-2-en-6-carbonsäure-2-(acryloyloxy)-äthylester wird in Form eines etwa 20 µm dicken Überzugs 2 Sekunden lang wie in der ersten Stufe von Beispiel 1 bestrahlt, wobei er klebfrei wird. Danach bestrahlt man ihn 15 Minuten lang wie in der zweiten Stufe, wobei eine sichtbare Abbildung entsteht.

*Beispiel 3*

Eine Zusammensetzung aus 10 Teilen 5-Carboxy-7-oxabicyclo[2.2.1]hept-2-en-6-carbonsäure-2-(acryloyloxy)-äthylester und 1 Teil Benzyldimethylketal wird 15 Sekunden lang wie in der ersten Stufe von Beispiel 1 bestrahlt, wobei sie klebfrei wird. Danach bestrahlt man sie 15 Minuten lang mit einer Mitteldruckquecksilberlampe (80 W pro cm) durch ein Negativ hindurch, wobei eine sichtbare Abbildung entsteht.

*Beispiel 4*

Eine Zusammensetzung aus Methyl-5-carboxybicyclo[2.2.1]hept-2-en-6-carbonsäure-2-(acryloyloxy)-äthylester (30 Teile), 3-(Methacryloyloxy)-2-hydroxypropyl-X,Y-dibrom-p-kresyläther (30 Teile), N-(2-Methacryloyloxyäthyl)-2,3-dimethylmaleinimid (8 Teile), Benzyldimethylketal (8 Teile) und 2-Chlorthioxanthon (2 Teile) wird gemäss der ersten Stufe von Beispiel 1 15 Sekunden lang bestrahlt. Danach bestrahlt man den Überzug wie in der zweiten Stufe jenes Beispiels 15 Minuten lang durch ein Negativ hindurch und entwickelt dann in 1m-Natronlauge. Es ensteht eine ausgezeichnete Reliefabbildung.

*Beispiel 5*

Ein Gemisch aus 45 Teilen Methyl-5-carboxybicyclo[2.2.1]hept-2-en-6-carbonsäure-2-(methacryloyloxy)-äthylester und 45 Teilen Methyl-5-carbomethoxybicyclo[2.2.1]hept-2-en-6-carbonsäure-3-(methacryloyloxy)-2-hydroxypropylester, das 8 Teile Benzyldimethylketal und 2 Teile 2-Chlorthioxanthon enthält, wird in Form einer etwa 20 µm dicken Schicht im Abstand von 20 cm mit einer

Mitteldruckquecksilberlampe (80 W pro cm) bestrahlt, wobei die Schicht nach 10 bis 15 Sekunden klebfrei wird. Danach bestrahlt man den Überzug 10 Minuten lang durch ein Negativ hindurch mit einer Mitteldruckquecksilberlampe (30 W pro cm) im Abstand von 25 cm und entwickelt dann mit 0,1m wässriger Natronlauge. Es entsteht eine Abbildung.

*Beispiel 6*

In ein Gemisch aus 60 Teilen N-(2-Methacryloyloxyäthyl)-methylbicyclo[2.2.1]hept-2-en-5,6-dicarboximid und 30 Teilen Methyl-5-carboxybicyclo-[2.2.1]hept-2-en-6-carbonsäure-2-(methacryloyloxy)-äthylester werden 4 Teile Benzyldimethylketal und 4 Teile 2-Chlorthioxanthon eingearbeitet, indem man diese in einer kleinen Menge Aceton gelöst dazugibt und zwecks Entfernung des Lösungsmittels kurz auf 50°C erwärmt. Man verfährt mit der Zusammensetzung wie in Beispiel 5, ausser dass nur 3 Sekunden Belichtung mit der Strahlung erforderlich sind, um die Schicht klebfrei zu machen. Nach weiterer 30 Minuten langer Belichtung erhält man bei der Entwicklung Bürsten mit 1%iger wässriger Natriumcarbonatlösung eine ausgezeichnete Abbildung.

*Beispiel 7*

Man vermischt 3-(Methacryloyloxy)-2-hydroxypropyl-X,Y-dibrom-p-kresyläther (66 Teile) und 5--Carboxy-7-oxabicyclo[2.2.1]hept-2-en-6-carbonsäure-2-(methacryloyloxy)-äthylester (33 Teile) und arbeitet Benzildimethylketal (4 Teile) und 2-Chlorthioxanthon (4 Teile) ein. Man verfährt mit der Zusammensetzung wie in Beispiel 5, ausser dass nur 5 Sekunden Belichtung mit Strahlung erforderlich sind, um die Schicht klebfrei zu machen. Nach weiterer 30 Minuten langer Belichtung erhält man bei der Entwicklung durch Bürsten mit 0,1%iger wässriger Natriumcarbonatlösung eine gute Abbildung.

*Beispiel 8*

Man vereinigt Methyl-5-carboxybicyclo[2.2.1]-hept-2-en-6-carbonsäure-2-(methacryloyloxy)--äthylester (90 Teile) und Methyl-5-carbomethoxybicyclo[2.2.1]hept-2-en-6-carbonsäure-3-(methacryloyloxy)-2-hydroxypropylester (90 Teile) und vermischt mit Benzyldimethylketal (16 Teile) und 2-Chlorthioxanthon (4 Teile). Diese Zusammensetzung wird als etwa 20 µm dicke Schicht auf eine Kupferplatte aufgebracht und mit einer Mitteldruckquecksilberlampe (80 W pro cm) im Abstand von 20 cm bestrahlt, wobei die Schicht nach 10 Sekunden klebfrei wird.

Die beschichtete Platte wird dann bei Raumtemperatur 14 Tage im Dunkeln gelagert, wonach man die Beschichtung durch ein Negativ hindurch 60 Minuten lang mit einer Mitteldruckquecksilberlampe (30 W pro cm) im Abstand von 25 cm bestrahlt. Entwickeln mit 2%igem Natriumcarbonat ergibt eine dauerhafte Abbildung.

*Vergleichsbeispiel*

Nur zur Vergleichszwecken belichtet man eine Zusammensetzung aus 45 Teilen Methylbicyclo[2.2.1]-hept-2-en-5,6-dicarbonsäureanhydrid, 45 Teilen Methacrylsäure, 8 Teilen Benzyldimethylketal und 2 Teilen 2-Chlorthioxanthon in Form eines Films mit der Strahlung einer Mitteldruckquecksilberlampe (80 W pro cm). Nach längerer Bestrahlung (mehr als 30 Sekunden) war diese Zusammensetzung, die Gruppen der Formel I und Gruppen der Formel II in getrennten Molekülen enthält, noch nicht klebfrei.

**Patentansprüche**

1. Verfahren zur Erzeugung einer Abbildung, wobei man (1) eine auf einem Träger aufgebrachte Schicht aus einer flüssigen Zusammensetzung so mit aktinischer Strahlung belichtet, dass sich die Schicht durch Photopolymerisation verfestigt und im wesentlichen klebfrei wird, jedoch weitgehend photovernetzbar bleibt, und anschliessend (2) die so verfestigte Schicht durch eine bildtragende Vorlage mit weitgehend undurchsichtigen und weitgehend durchsichtigen Stellen hindurch mit einer wesentlich grösseren Menge aktinischer Strahlung belichtet, so dass die weiterbelichtete(n) Stelle(n) der photopolymerisierten Schicht erneut Photovernetzung eingehen und (3) die Abbildung durch Auflösung der nicht weitgehend photovernetzten Stellen der Schicht in einem Lösungsmittel entwickelt, dadurch gekennzeichnet, dass man eine flüssige Zusammensetzung verwendet, die eine Verbindung (A) mit sowohl mindestens einer Gruppe der Formel I

$$CH_2=\underset{\displaystyle R}{\underset{|}{C}}CO- \qquad \text{I}$$

als auch mindestens einer Gruppe der Formel II

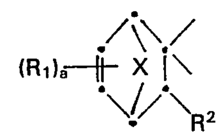

II

im selben Molekül enthält, wobei R ein Wasserstoffatom oder eine Methylgruppe bedeutet, a null oder eine ganze Zahl von 1 bis 4 ist, $R^1$ eine Methylgruppe darstellt oder, falls a 1 ist, auch für eine Allylgruppe stehen kann, $R^2$ ein Wasserstoffatom, eine Carboxylgruppe, eine gegebenenfalls in der Kette durch ein Äthersauerstoffatom unterbrochene Carbonyloxyalkylgruppe, eine über ein Sauerstoffatom an eine Gruppe der Formel I gebundene Carbonyloxyalkylengruppe, wobei die Alkyl- bzw. Alkylengruppe jeweils bis zu 8 Kohlenstoffatome aufweist, oder zusammen mit einer der angegebenen freien Valenzen eine cyclische Imidgruppe der Konstitution $\begin{matrix}-OC\\-OC\end{matrix}\!\!>N-$ darstellt und X entweder eine gegebenenfalls durch eine oder zwei der oben erwähnten Methylgruppen $R^1$ oder durch die Allylgruppe $R^1$ substituierte Methylenbrücke oder ein Sauerstoffatom bedeutet, wobei die Photopolymerisation von (A) in Verfahrensschritt (1) über die Gruppe bzw. Gruppen der Formel I und die anschliessende Photovernetzung in Verfahrensschritt (2) über die Gruppe bzw. Gruppen der Formel II erfolgt.

2. Verfahren nach Anspruch 1, dadurch gekennzeichnet, dass (A) der Formel

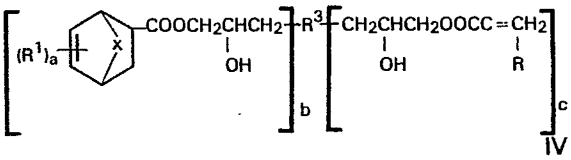

IV

entspricht, worin b und c unabhängig voneinander je eine ganze Zahl von mindestens 1 sind, R, $R^1$, X und a die in Anspruch 1 angegebenen Bedeutungen haben und $R^3$ den Rest einer Verbindung mit mindestens (b + c) direkt an ein bzw. mehrere Sauerstoff-, Stickstoff- oder Schwefelatom(e) gebundenen Glycidylgruppen nach Wegnahme von (b + c) solchen Glycidylgruppen darstellt.

3. Verfahren nach Anspruch 1, dadurch gekennzeichnet, dass (A) der Formel

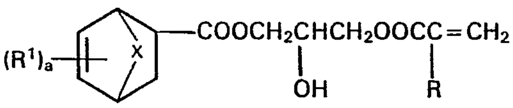

VII

oder

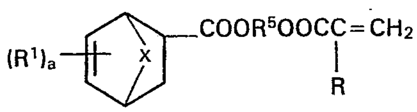

XII

entspricht, worin R, $R^1$, X und a die in Anspruch 1 angegebenen Bedeutungen haben und $R^5$ eine Alkylengruppe mit 2 bis 6 Kohlenstoffatomen darstellt.

4. Verfahren nach Anspruch 1, dadurch gekennzeichnet, dass (A) der Formel

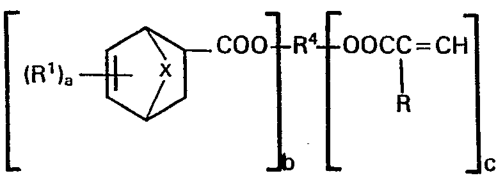

IX

entspricht, worin R, $R^1$, X und a die in Anspruch 1 sowie b und c die in Anspruch 2 angegebenen Bedeutungen haben und $R^4$ den Rest eines Alkohols mit mindestens (b + c) alkoholischen Hydroxylgruppen bzw. eines Phenols mit mindestens (b + c) phenolischen Hydroxylgruppen nach Wegnahme von (b + c) alkoholischen bzw. phenolischen Hydroxylgruppen darstellt.

5. Verfahren nach Anspruch 1, dadurch gekennzeichnet, dass (A) der Formel

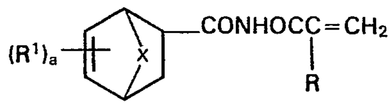

XIV

oder

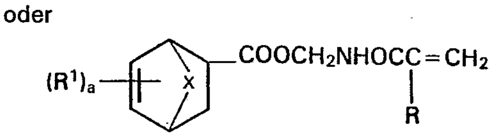

XVI

oder

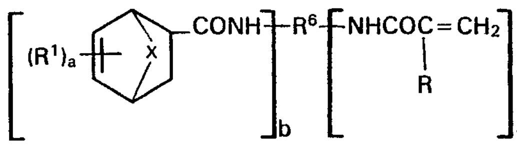

XVIII

entspricht, worin R, $R^1$, X und a die in Anspruch 1 sowie b und c die in Anspruch 2 angegebenen Bedeutungen haben und $R^6$ den Rest einer Verbindung mit mindestens (b + c) primären Aminogruppen nach Wegnahme von (b + c) solchen Aminogruppen darstellt.

6. Verfahren nach Anspruch 1, dadurch gekennzeichnet, dass (A) der Formel

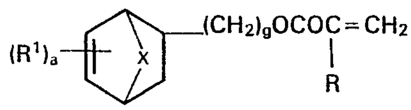

XVII

oder

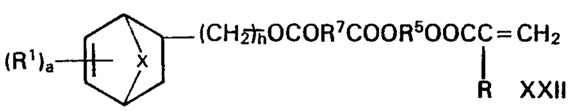

XXII

oder

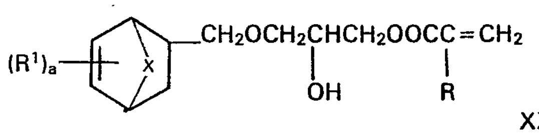

XXV

oder

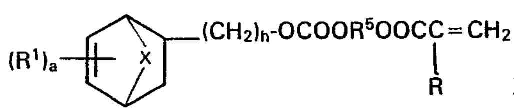

XXVI

entspricht, worin R, $R^1$, X und a die in Anspruch 1 und $R^5$ die in Anspruch 3 angegebenen Bedeutungen haben, $R^7$ eine Kohlenstoff-Kohlenstoffbindung oder eine zweiwertige Gruppe mit 1 bis 10 Kohlenstoffatomen darstellt sowie g null oder 1 und h null, 1 oder 2 sind.

7. Verfahren nach Anspruch 1, dadurch gekennzeichnet, dass (A) der Formel

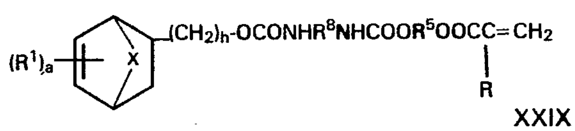

XXIX

entspricht, worin R, $R^1$, X und a die in Anspruch 1, $R^5$ die in Anspruch 3, h die in Anspruch 6 angegebenen Bedeutungen haben und $R^8$ den Rest eines organischen Diisocyanats nach Wegnahme der beiden Isocyanatgruppen darstellt.

8. Verfahren nach Anspruch 1, dadurch gekennzeichnet, dass (A) der Formel

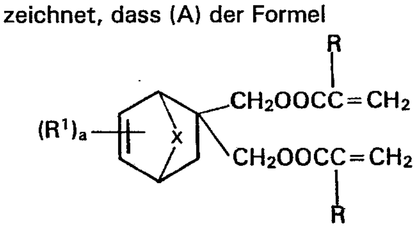

XXXII

oder

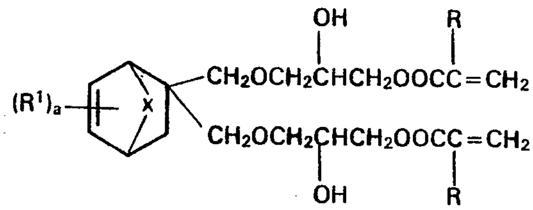

XXXIII

entspricht, worin R, $R^1$, X, und a die in Anspruch 1 angegebenen Bedeutungen haben.

9. Verfahren nach Anspruch 1, dadurch gekennzeichnet, dass (A) der Formel

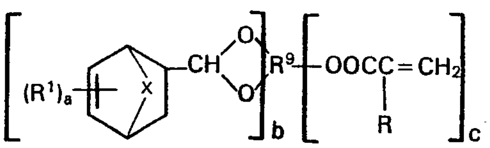

XXXIV

entspricht, worin R, $R^1$, X und a die in Anspruch 1 sowie b und c die in Anspruch 2 angegebenen Bedeutungen haben und $R^9$ den Rest einer mindestens (2b + c) alkoholischen Hydroxylgruppen enthaltenden Verbindung nach Wegnahme von (2b + c) alkoholischen Hydroxylgruppen darstellt.

10. Verfahren nach Anspruch 1, dadurch gekennzeichnet, dass (A) der Formel

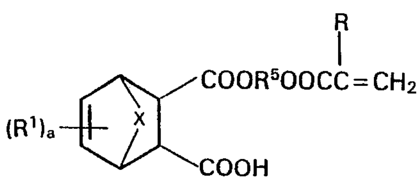

XXXIX

oder

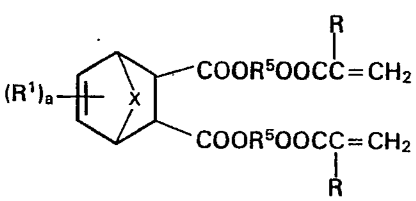

XL

entspricht, worin R, $R^1$, X und a die in Anspruch 1 und $R^5$ die in Anspruch 3 angegebenen Bedeutungen haben.

11. Verfahren nach Anspruch 1, dadurch gekennzeichnet, dass (A) der Formel

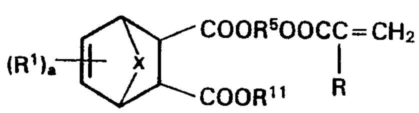

XLI

oder

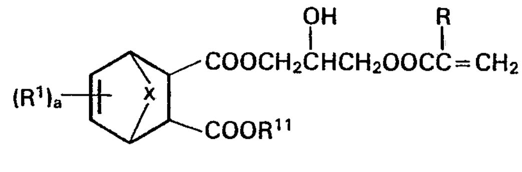

XLII

entspricht, worin R, $R^1$, X und a die in Anspruch 1 und $R^5$ die in Anspruch 3 angegebenen Bedeutungen haben und $R^{11}$ eine gegebenenfalls in der Kette durch ein Äthersauerstoffatom unterbrochene Alkylgruppe mit 1 bis 8 Kohlenstoffatomen darstellt.

12. Verfahren nach Anspruch 1, dadurch gekennzeichnet, dass (A) der Formel

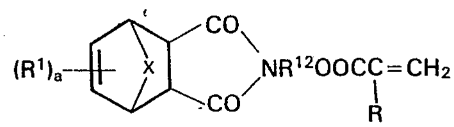

XLIII

entspricht, worin R, $R^1$, X und a die in Anspruch 1 angegebenen Bedeutungen haben und $R^{12}$ eine Alkylengruppe mit 1 bis 6 Kohlenstoffatomen darstellt.

13. Verfahren nach Anspruch 1, dadurch gekennzeichnet, dass als (A) Bicyclo[2.2.1]hept-2-en-6-carbonsäure-3-(methacryloyloxy)-2-hydroxypropylester, Methyl-5-carboxybicyclo[2.2.1]hept-2-en-6-carbonsäure-2-(acryloyloxy)-äthylester, 5-Carboxy-7-oxabicyclo[2.2.1]hept-2-en-6-carbonsäure-2-(acryloyloxy)-äthylester, Methyl-5-carboxybicyclo[2.2.1]hept-2-en-6-carbonsäure-2-(methacryloyloxy)-äthylester, Methyl-5-carbomethoxybicyclo[2.2.1]hept-2-en-6-carbonsäure-3-(methacryloyloxy)-2-hydroxypropylester, 5-Carboxy-7-oxabicyclo[2.2.1]hept-2-en-6-carbonsäure-2-(methacryloyloxy)-äthylester oder N-(2-Methacryloyloxyäthyl)-methylbicyclo[2.2.1]hept-2-en-5,6-dicarboximid vorliegt.

14. Verfahren nach Anspruch 1, dadurch gekennzeichnet, dass die Zusammensetzung eine weitere Verbindung der im Anspruch 10 definierten Formel XXXIX und/oder eine Verbindung (B) mit sowohl mindestens einer Gruppe der Formel I als auch mindestens einer freien Sulfon-, Phosphon- oder Carbonsäuregruppe, aber keiner Gruppe der Formel II im Molekül enthält, und dass als Lösungsmittel zum Entwickeln der Abbildung eine wässrige Lösung einer Base verwendet wird.

15. Verfahren nach Anspruch 1, dadurch gekennzeichnet, dass diese flüssige Zusammensetzung ferner eine Verbindung (B) mit sowohl mindestens einer Gruppe der Formel I und mindestens einer primären, sekundären oder tertiären Amingruppe, aber keiner Gruppe der Formel II im Molekül enthält, und dass als Lösungsmittel zum Entwickeln der Abbildung eine wässrige Lösung einer Säure verwendet wird.

**Claims**

1. A process for the production of an image by (1) exposing to actinic radiation a layer, supported on a carrier, of a liquid composition such that the layer solidifies and becomes essentially nontacky due to

photopolymerisation but remains substantially photocrosslinkable, and subsequently (2) exposing through an image-bearing transparency consisting of substantially opaque and substantially transparent areas the layer thus solidifies to a substantially greater amount of actinic radiation such that the further exposed part or parts of the photopolymerised layer undergo photocrosslinking again, and (3) developing the image by dissolving in a solvent parts of the layer which have not become substantially photocrosslinked, which process comprises using a liquid composition containing a compound (A) having in the same molecule both at least one group of the formula I

CH2=CCO-
|
R
I

and at least one group of formula II

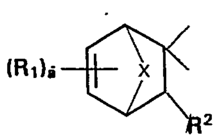
II

in which formulae R is a hydrogen atom or a methyl group, a is zero or an integer from 1 to 4, $R^1$ is a methyl group, or, if a is 1, $R^1$ may alternatively be an allyl group, $R^2$ is a hydrogen atom, a carboxyl group, or is a carbonyloxyalkyl group which may be interrupted in the chain by an ether oxygen atom, or $R^2$ is a carbonyloxyalkylene group attached through an oxygen atom to a group of formula I, the alkyl or alkylene group having up to 8 carbon atoms each, or together with one of the indicated free valencies, $R^2$ is a cyclic imide group of structure 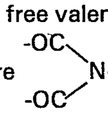, and X is a methylene bridge, a methylene bridge substituted by one or two of the aforesaid methyl groups $R^1$, a methylene bridge substituted by the allyl group $R^1$, or X is an oxygen atom, with the photopolymerisation of (A) in process step (1) being effected via the group or groups of formula I and the subsequent photocrosslinking in process step (2) being effected via the group or groups of formula II.

2. A process according to claim 1, wherein (A) is of the formula

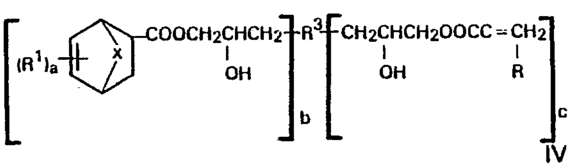
IV

wherein b and c are each independently an integer of at least 1, R, $R^1$, X and a are as defined in claim 1, and $R^3$ is the residue of a compound containing at least (b+c) glycidyl groups directly attached to one or more oxygen, nitrogen, or sulfur atoms, after removal of (b+c) such glycidyl groups.

3. A process according to claim 1, wherein (A) is of the formula

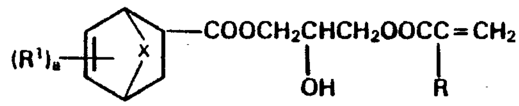
VII

or

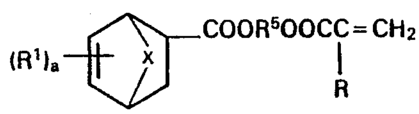
XII

in which formulae R, $R^1$, X and a are as defined in claim 1, and $R^5$ is an alkylene group of 2 to 6 carbon atoms.

4. A process according to claim 1, wherein (A) is of the formula

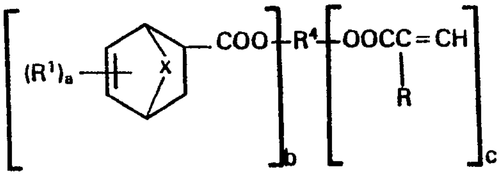
IX

wherein R, $R^1$, X and a are as defined in claim 1, b and c are as defined in claim 2, and $R^4$ is the residue of an alcohol having at least (b+c) alcoholic hydroxyl groups or of a phenol having at least (b+c) phenolic hydroxyl groups, after removal of (b+c) alcoholic hydroxyl or phenolic hydroxyl groups.

5. A process according to claim 1, wherein (A) is of the formula

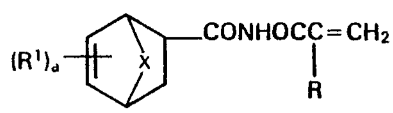
XIV

or

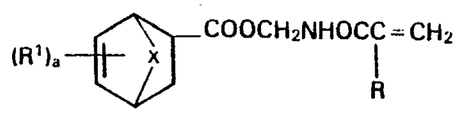
XVI

or

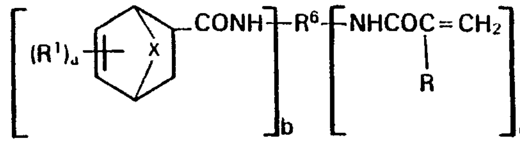
XVIII

in which formulae R, $R^1$, X and a are as defined in claim 1, b and c are as defined in claim 2, and $R^6$ is the residue of a compound containing at least (b+c) primary amino groups, after removal of (b+c) such amino groups.

6. A process according to claim1, wherein (A) is of the formula

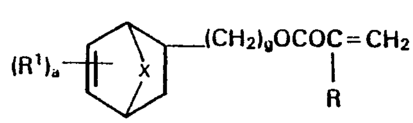
XVII

or

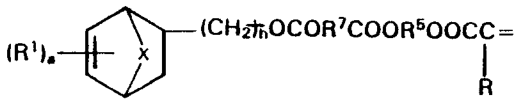

XXII

or

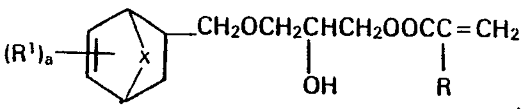

XXV

or

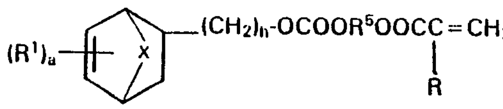

XXVI

in which formulae R, $R^1$, X and a are as defined in claim 1, $R^5$ is as defined in claim 3, $R^7$ is a carbon-carbon bond or a divalent group of 1 to 10 carbon atoms, g is zero or 1, and h is zero, 1 or 2.

7. A process according to claim 1, wherein (A) is of the formula

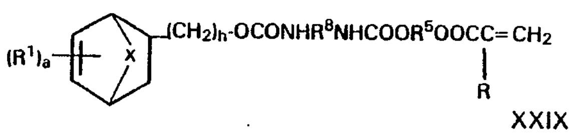

XXIX

wherein R, $R^1$, X and a are as defined in claim 1, $R^5$ is as defined in claim 3, h is as defined in claim 6, and $R^8$ is the residue of an organic diisocyanate, after removal of the two isocyanate groups.

8. A process according to claim 1, wherein (A) is of the formula

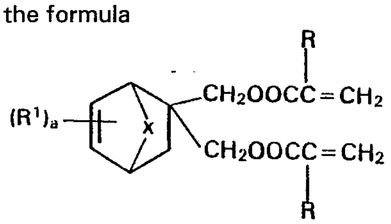

XXXII

or

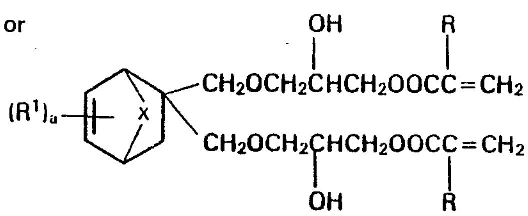

XXXIII

in which formulae R, $R^1$, X and a are as defined in claim 1.

9. A process according to claim 1, wherein (A) is of the formula

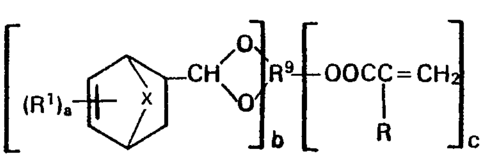

XXXIV

wherein R, $R^1$, X and a are as defined in claim 1, b and c are as defined in claim 2, and $R^9$ is the residue of a compound containing at least (2b + c) alcoholic hydroxyl groups, after removal of (2b + c) alcoholic hydroxyl groups.

10. A process according to claim 1, wherein (A) is of the formula

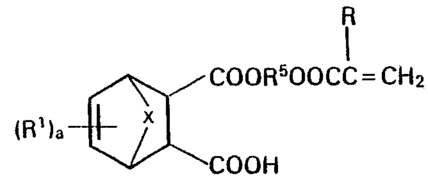

XXXIX

or

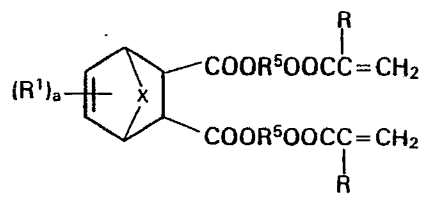

XL

in which formulae R, $R^1$, X and a are as defined in claim 1, and $R^5$ is as defined in claim 3.

11. A process according to claim 1, wherein (A) is of the formula

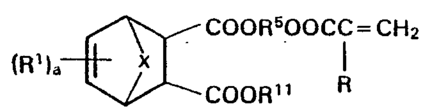

XLI

or

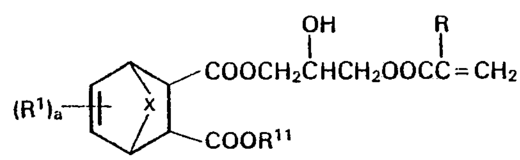

XLII

in which formulae R, $R^1$, X and a are as defined in claim 1, $R^5$ is as defined in claim 3, and $R^{11}$ is an alkyl group of 1 to 8 carbon atoms, or an alkyl group of 1 to 9 carbon atoms interrupted in the chain by an ether oxygen atom.

12. A process according to claim 1, wherein (A) is of the formula

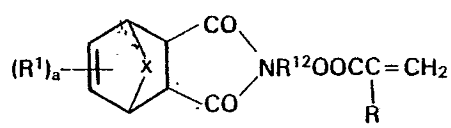

XLIII

wherein R, $R^1$, X and a are as defined in claim 1, and $R^{12}$ is an alkylene group of 1 to 6 carbon atoms.

13. A process according to claim 1, wherein (A) is 3-(methacryloyloxy)-2-hydroxypropyl bicyclo[2.2.-1]hept-2-ene-6-carboxylate, 2-(acryloyloxy)ethyl methyl-5-carboxy-bicyclo[2.2.1]hept-2-ene-6-carboxylate, 2-(acryloyloxy)ethyl 5-carboxy-7-oxa-bicyclo[2.2.1]hept-2-ene-6-carboxylate, 2-(meth-acryloyloxy)ethyl methyl-5-carboxybicyclo[2.2.1]-hept-2-ene-6-carboxylate, 3-(methacryloyloxy)-2--hydroxypropyl methyl-5-carbomethoxybicyclo-[2.2.1]hept-2-ene-6-carboxylate, 2-(methacryloyl-oxy)ethyl 5-carboxy-7-oxabicyclo[2.2.1]hept-2--ene-6-carboxylate, or N-2-(methacryloyloxy)ethyl methylbicyclo[2.2.1]hept-2-ene-5,6-dicarboximide.

14. A process according to claim 1, wherein the liquid composition contains a further compound of formula XXXIX as defined in claim 10 and/or a compound (B) which has in the molecule both at least one group of formula I and at least one free sulfonic, phosphonic, or carboxylic acid group but has no group of formula II, and the solvent employed for development of the image is an aqueous solution of a base.

15. A process according to claim 1, wherein the liquid composition also contains a compound (B) which has in the molecule both at least one group of formula I and at least one primary, secondary, or tertiary amino group but has no group of formula II, and the solvent employed for development of the image is an aqueous solution of an acid.

**Revendications**

1. Procédé pour créer une image selon lequel (1) une couche d'une composition liquide, appliquée sur un support, est exposée à un rayonnement actinique de telle façon que ladite couche se solidifie par photopolymérisation et devienne pratiquement non collante mais tout en restant dans une large mesure photoréticulable, puis (2) la couche ainsi solidifiée est exposée, à travers un original comportant des endroits pratiquement opaques et des endroits pratiquement transparents, à une quantité de rayonnement actinique beaucoup plus grande de telle façon que le ou les endroits de la couche photopolymérisée qui ont ainsi reçu une quantité supplémentaire de rayonnement subissent à nouveau une photoréticulation, et (3) l'image est développée par dissolution, dans un solvant, des endroits de la couche qui n'ont pas subi une photoréticulation poussée, le procédé en question étant caractérisé en ce qu'on utilise une composition liquide renfermant un composé (A) dans la molécule duquel il y a à la fois au moins un radical de formule I

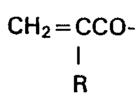

I

et au moins un radical de formule II

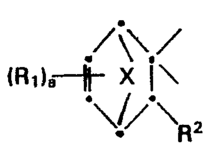

II

formules dans lesquelles: R représente un atome d'hydrogène ou un radical méthyle, a représente un entier de 0 à 4, $R^1$ représente un radical méthyle ou, dans le cas où a est égal à 1, un radical allyle, $R^2$ représente un atome d'hydrogène, un radical carboxy, un radical carbonyloxy-alkyle (dont la chaîne est éventuellement interrompue par un atome d'oxygène de fonction éther), un radical R carbonyloxy-alkylène relié à un radical de formule I par un atome d'oxygène, l'alkyle et l'alkylène contenant chacun au plus 8 atomes de carbone, ou forme, avec l'une des valences libres représentées, un radical d'imide cyclique ayant la constitution suivante:

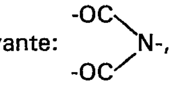

et X représente soit un pont méthylène (éventuellement porteur d'un ou de deux des radicaux méthyles $R^1$ mentionnés ci-dessus ou d'un radical allyle $R^1$) ou un atome d'oxygène, la photopolymérisation de (A) dans l'étape opératoire (1) se faisant par le ou les radicaux de formule I, et la photoréticulation ultérieure, dans l'étape opératoire (2), se faisant par le ou les radicaux de formule II.

2. Procédé selon la revendication 1, caractérisé en ce que le composé (A) répond à la formule IV

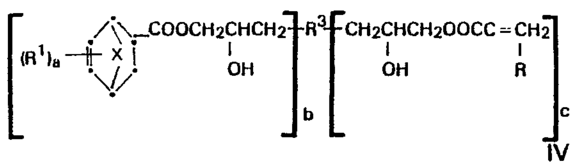

IV

dans laquelle b et c représentent chacun, indépendamment l'un de l'autre, un nombre entier au moins égal à 1, R, $R^1$, X et a ont les significations données à la revendication 1, et $R^3$ représente le radical qui subsiste lorsque, d'un composé contenant au moins (b + c) radicaux glycidyles directement liés à un ou à plusieurs atomes d'oxygène, d'azote ou de soufre, on a retiré (b + c) radicaux de ce genre.

3. Procédé selon la revendication 1, caractérisé en ce que le composé (A) répond à l'une des formules VII et XII

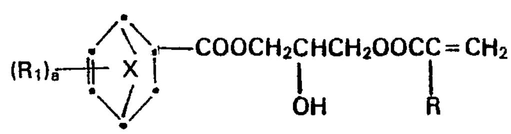

VII

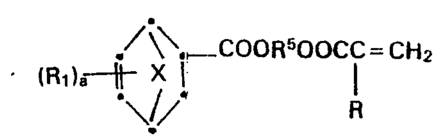

XII

dans lesquelles R, $R^1$, X et a ont les significations données à la revendication 1 et $R^5$ représente un radical alkylène contenant de 2 à 6 atomes de carbone.

4. Procédé selon la revendication 1, caractérisé en ce que le composé (A) répond à la formule IX

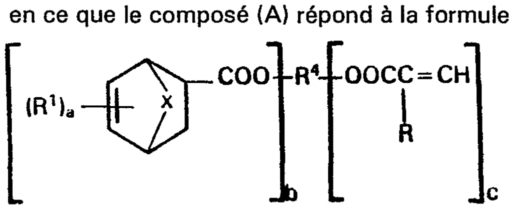

IX

dans laquelle R, $R^1$, X et a ont les significations données à la revendication 1, b et c ont les significations données à la revendication 2 et $R^4$ représente le

radical qui reste lorsque, d'un alcool contenant au moins (b+c) radicaux hydroxy alcooliques ou d'un phénol contenant au moins (b+c) radicaux hydroxy phénoliques, on a retiré (b+c) radicaux hydroxy alcooliques ou phénoliques de ce genre.

5. Procédé selon la revendication 1, caractérisé en ce que le composé (A) répond à l'une des formules suivantes

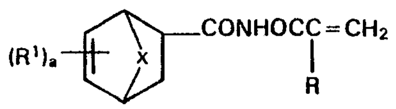
XIV

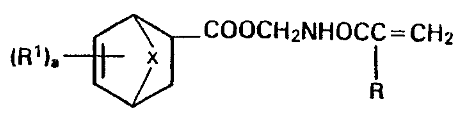
XVI

et

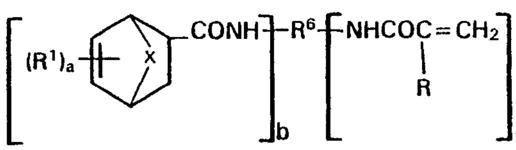
XVIII

dans lesquelles R, $R^1$, X et a ont les significations données à la revendication 1, b et c ont les significations données à la revendication 2, et $R^6$ représente le radical qui reste lorsque, d'un composé contenant au moins (b+c) radicaux amino primaires, on a enlevé (b+c) radicaux amino de ce genre.

6. Procédé selon la revendication 1, caractérisé en ce que le composé (A) répond à l'une des formules suivantes

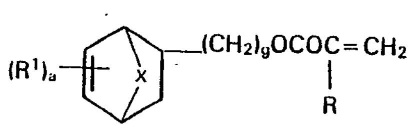
XVII

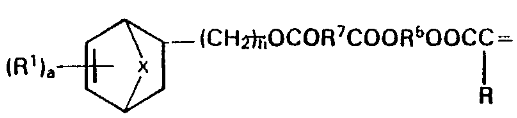
XXII

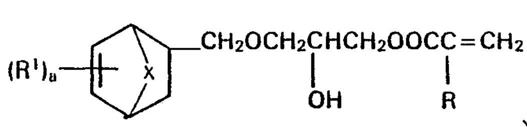
XXV

et

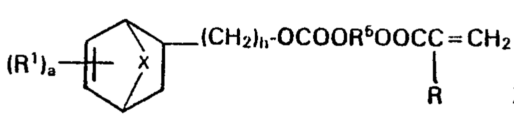
XXVI

dans lesquelles R, $R^1$, X et a ont les significations données à la revendication 1, $R^5$ a la signification donnée à la revendication 3, $R^7$ représente une liaison carbone-carbone ou un radical bivalent contenant de 1 à 10 atomes de carbone, g est égal à 0 ou à 1 et h est égal à 0, à 1 ou à 2.

7. Procédé selon la revendication 1, caractérisé en ce que le composé (A) répond à la formule XXIX

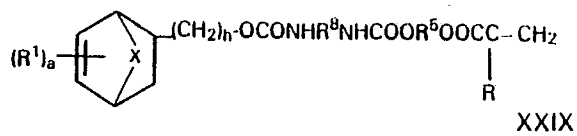
XXIX

dans laquelle R, $R^1$, X et a ont les significations données à la revendication 1, $R^5$ a la signification donnée à la revendication 3, h a la signification donnée à la revendication 6, et $R^8$ représente le radical d'un di-isocyanate organique que l'on a amputé de ses deux radicaux isocyanato.

8. Procédé selon la revendication 1, caractérisé en ce que le composé (A) répond à l'une des formules XXXII et XXXIII

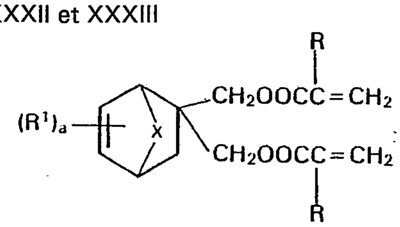
XXXII

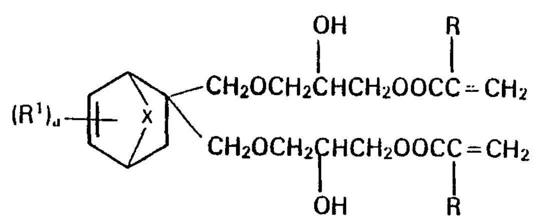
XXXIII

dans lesquelles R, $R^1$, X et a ont les significations données à la revendication 1.

9. Procédé selon la revendication 1, caractérisé en ce que le composé (A) répond à la formule XXXIV

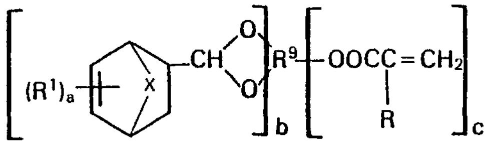
XXXIV

dans laquelle R, $R^1$, X et a ont les significations données à la revendication 1, b et c ont les significations données à la revendication 2, et $R^9$ représente le radical qui reste lorsque, d'un composé contenant au moins (2b+c) radicaux hydroxy alcooliques, on a retiré (2b+c) radicaux hydroxy alcooliques.

10. Procédé selon la revendication 1, caractérisé en ce que le composé (A) répond à l'une des formules XXXIX et XL

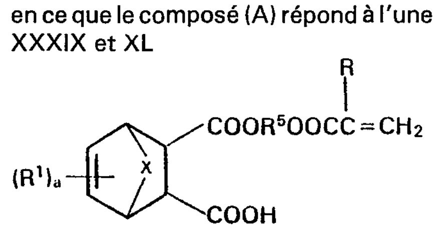
XXXIX

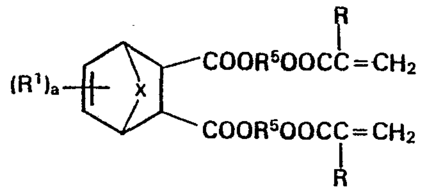

XL ;

dans lesquelles R, $R^1$, X et a ont les significations données à la revendication1 et $R^5$ a la signification donnée à la revendication 3.

11. Procédé selon la revendication 1, caractérisé en ce que le composé (A) répond à l'une des formules XLI et XLII

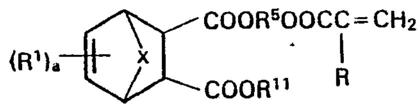

XLI

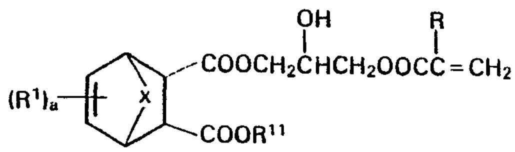

XLII

dans lesquelles R, $R^1$, X et a ont les significations données à la revendication1, $R^5$ a la signification donnée à la revendication 3 et $R^{11}$ représente un radical alkyle qui contient de 1 à 8 atomes de carbone et dont la chaîne est éventuellement interrompue par un atome d'oxygène de fonction éther.

12. Procédé selon la revendication 1, caractérisé en ce que le composé (A) répond à la formule XLIII

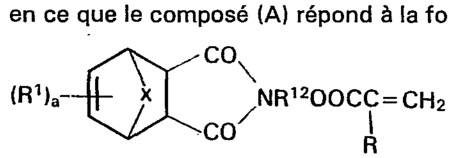

XLIII

dans laquelle R, $R^1$, X et a ont les significations données à la revendication1 et $R^{12}$ représente un radical alkylène contenant de 1 à 6 atomes de carbone.

13. Procédé selon la revendication 1, caractérisé en ce que le composé (A) est le bicyclo[2.2.1]heptène-2 carboxylate-6 de méthacryloyloxy-3 hydroxy-2 propyle, l'ester acryloyloxy-2 éthylique de l'acide méthyl carboxy-5 bicyclo[2.2.1]heptène-2 carboxylique-6, l'ester acryloyloxy-2 éthylique de l'acide de carboxy-5 oxa-7 bicyclo[2.2.1]heptène-2 carboxylique-6, l'ester méthacryloyloxy-2 éthylique de l''acide méthyl carboxy-5 bicyclo[2.2.1]heptène-2 carboxylique-6, le méthyl méthoxycarbonyl-5 bicyclo[2.2.1]heptène-2 carboxylate-6 de méthacryloyloxy-3 hydxroxy-2 propyle, l'ester méthacryloyloxy-2 éthylique de l'acide carboxy-5 oxa-7 bicyclo-[2.2.1]heptène-2 carboxylique-6 ou le N-(méthacryloyloxy-2 éthyl)-imide de l'acide méthyl-bicyclo-[2.2.1]heptène-2 dicarboxylique-5,6.

14. Procédé selon la revendication 1, caractérisé en ce que la composition contient un autre composé répondant à la formule XXXIX définie à la revendication 10 et/ou un composé (B) dans la molécule duquel il y a à la fois au moins un radical de formule I et au moins un radical d'acide sulfonique, phosphonique ou carboxylique libre mais pas de radical de formule II, et en ce qu'on utilise, comme solvant pour développer l'image, une solution aqueuse d'une base.

15. Procédé selon la revendication 1, caractérisé en ce que cette composition liquide renferme en outre un composé (B) dont la molécule contient à la fois au moins un radical de formule I et au moins un radical amino primaire, secondaire ou tertiaire mais pas de radical de formule II, et en ce qu'on utilise, comme solvant pour développer l'image, une solution aqueuse d'un acide.